# EUROPÄISCHE PATENTSCHRIFT

(11) **EP 1 536 894 B1**
(45) Veröffentlichungstag und Bekanntmachung des Hinweises auf die Patenterteilung: **30.03.2011**
(21) Anmeldenummer: 03793758.8
(22) Anmeldetag: 26.08.2003
(51) Int. Cl.: B05B 11/02, B05B 11/00, A61M 15/00

(54) **APPARAT ZUM AUSBRINGEN VON FLUESSIGKEITEN, HIERZU GEEIGNETE BEHAELTERPATRONE, UND SYSTEM AUS DEM APPARAT ZUM AUSBRINGEN VON FLUESSIGKEITEN UND DER BEHAELTERPATRONE**
DEVICE FOR DISCHARGING LIQUIDS, A CARTRIDGE SUITED THEREFOR, AND SYSTEM COMPRISED OF THE DEVICE FOR DISCHARGING LIQUIDS AND OF THE CARTRIDGE
APPAREIL PERMETTANT D'EXPULSER DES LIQUIDES, CARTOUCHES ADAPTEES ET SYSTEME COMPOSE DE L'APPAREIL PERMETTANT D'EXPULSER LES LIQUIDES ET DE LA CARTOUCHE

(30) Priorität: 05.09.2002 DE 10241640; 03.12.2002 EP 02027030
(43) Veröffentlichungstag der Anmeldung: 08.06.2005
(73) Patentinhaber: Boehringer Ingelheim Pharma GmbH & Co.KG, 55216 Ingelheim am Rhein (DE)
(72) Erfinder: SCHIEWE, Joerg, 55129 Mainz (DE); ZIERENBERG, Bernd, 55411 Bingen (DE); WERGEN, Horst, 42105 Wuppertal (DE); WUTTKE, Gilbert, 44149 Dortmund (DE); DUNNE, Stephen, Suffolk IP 14 3 AE (GB)
(86) Internationale Anmeldenummer: PCT/EP2003/009442
(87) Internationale Veröffentlichungsnummer: WO 2004/022244

(56) Entgegenhaltungen:
- WO-A-00/47332
- WO-A-97/12687
- US-A- 4 623 337
- US-B1- 6 401 987

## Beschreibung

Die vorliegende Erfindung betrifft einen treibgasfreien Apparat zum Ausbringen von Flüssigkeiten, eine dazu passende Behälterpatrone zur Bevorratung der Flüssigkeit und das Ensemble aus beidem. Insbesondere besteht die Erfindung aus
a) einer Vorrichtung zur Beaufschlagung von Druck auf einen Vorratsbehälter (Behälterpatrone), welche Mittel zur Aufnahme des Vorratsbehälters aufweist und
b) dem Vorratsbehälter selbst, wobei in diesen eine Ausbringungseinrichtung für Flüssigkeit, z.B. in Form einer Düse und/oder Düseneinrichtung integriert ist.

Der erfindungsgemäße Apparat kann z.B. als nadelloser Injektor oder als Zerstäuber verwendet werden. Als Zerstäuber dient er zur Bereitstellung eines Aerosols aus Tröpfchen zur inhalativen Aufnahme durch den Mund- und Rachenbereich in die Lunge eines Patienten oder zur nasalen Aufnahme. Weiterhin kann der erfindungsgemäße Zerstäuber unter Zuhilfenahme eines ergänzenden Adapters auch für die Augenbehandlung eingesetzt werden.

Im Rahmen der vorliegenden Erfindungsbeschreibung wird der Begriff Apparat mit den Begriffen Gerät, nadelloser Injektor, Zerstäuber oder auch Dosierinhalationsgerät gleichgesetzt. Die Begriffe können gleichrangig nebeneinander verwendet werden. Je nach Kontext kann unter diesen Begriffen nur die Vorrichtung zur Beaufschlagung von Druck verstanden werden oder das Ensemble derselben zusammen mit der Behälterpatrone. Der Unterschied zwischen dem erfindungsgemäßen Zerstäuber und dem nadellosen Injektor besteht funktionell hauptsächlich in der Ausgestaltung der Ausbringungseinrichtung: Im Fall des nadellosen Injektors ist diese so konstruiert, daß daraus ein Flüssigkeitsstrahl austritt, der als solches erhalten bleibt. Im Fall des Zerstäubers ist die Ausbringungseinrichtung so konstruiert, daß daraus entweder ein Aerosol austritt und/oder wenigstens zwei aufeinander treffende Flüssigkeitsstrahlen austreten, die durch den gegenseitigen Aufeinanderprall in ein Aerosol zerstäubt werden. Bevorzugt dient der erfindungsgemäße Vernebler als Inhalator für flüssige pharmazeutische Wirkstoff-Formulierungen . Letztere sind bevorzugt treibgasfrei und die pharmakologisch aktiven Bestandteile sind bevorzugt in Wasser, in Wasser-Ethanol-Gemischen oder in anderen pharmakologisch verträglichen, nicht flüchtigen Flüssigkeiten gelöst oder suspendiert. Bevorzugt handelt es sich bei den Formulierungen um Lösungen auf Wasser und/oder Wasser-Ethanol-Basis.

Derartige Formulierungen führen bei der inhalativen Applikation zu einer optimalen Wirkstoffverteilung der Wirksubstanzen in der Lunge, wenn sie mittels dafür geeigneten Inhalatoren in lungengängige Aerosole überführt werden.

### Stand der Technik

WO A 47332 offenbart eine gattungsgemäße Vorrichtung , für fließfähige Medien, wobei die Medien - wie z. B. ein Arzneimittel - vordosiert sind.
Diese Vorrichtung enthält eine Behälterpatrone, die in eine Aufnahmekammer der Vorrichtung einführbar ist und mittels einer Axialbewegung in ihre Endposition eingebracht wird. Mittels einer fest installierten, durchgehenden Bohrung, die in die Behälterpatrone hineinragt, kann das fließfähige Medien mittels einer Auslöstaste und der zuvor mittels Federkraft aufgebauten Drucks, über eine Hülse abgegeben werden.

US 6 401 987 offenbart eine Vorrichtung zur Abgabe von Flüssigkeiten, wobei die jeweilige Menge aus einem Vorratsbehälter mittels einer, z. B. durch Federkraft, vorbestimmten Energiemenge sowie einem Spannwerk über eine Ausgabevorrichtung abgegeben werden.

US 4 623 337 offenbart eine Vorrichtung zur Applikation von Flüssigkeiten ins Auge. Ähnlich der in US 6 401 987 beschriebenen Vorrichtung wird Druck zur Abgase der Flüssigkeit mittels einer Feder aufgebaut, um diese aus einem Vorratsbehälter dosiert abgehen zu können. Der Vorratsbehälter ist - ähnlich WO-A-47 332 - in eine Vorrichtung einführbar und verfügbar.

Die internationale Patentanmeldung WO 91/14468 "Atomizing Device and Methods" oder auch die WO 97/12687 offenbaren eine Vorrichtung zur treibgasfreien Verabreichung einer dosierten Menge eines flüssigen Arzneimittels zur inhalativen Anwendung. Auf die genannten Referenzen wird hiermit ausdrücklich Bezug genommen und die dort beschriebene Technologie wird im Rahmen der vorliegenden Erfindung als "Respimat^{®}-Technologie" bezeichnet. Unter diesem Begriff wird dabei insbesondere die Technologie verstanden, die einem Gerät gemäß den Figuren 6a und 6b der WO 97/12687 und der dazugehörigen Beschreibung prinzipiell zugrunde liegt, insbesondere die Technologie zur Druckbeaufschlagung, das Sperrspannwerk und die Mittel zur Ausbringung der Flüssigkeit

(Düse). Diese Inhalatoren können bereits Mengen von weniger als 100 Mikroliter einer flüssigen Wirkstofflösung in der therapeutisch notwendigen Dosierung binnen weniger Sekunden mit bevorzugt einem einzigen Hub in ein therapeutisch-inhalativ geeignetes Aerosol vernebeln. Dieses besteht aus Teilchen einer durchschnittlichen Größe von weniger als 20 Mikrometern. Der inhalierbare Anteil des Aerosols entspricht dabei der therapeutisch wirksamen Menge.
In diesen Verneblern auf Basis der Respimat^{®}-Technologie wird eine Arzneimittellösung mittels hohen Drucks von bis zu 500/600 bar in ein lungengängiges Aerosol überführt und versprüht. Dabei werden die Lösungsformulierungen in einem Reservoir gelagert. Von dort aus werden sie über ein Steigrohr in eine Druckkammer befördert und weiter über eine Düse vernebelt. Dabei ist es notwendig, daß die verwendeten Wirkstoff-Formulierungen eine ausreichende Lagerstabilität aufweisen und gleichzeitig so beschaffen sind, daß sie dem medizinischen Zweck entsprechend möglichst ohne weitere Manipulation, direkt appliziert werden können. Ferner dürfen sie keine Bestandteile aufweisen, die so mit dem Inhalator wechselwirken können, daß der Inhalator oder die pharmazeutische Qualität der Lösung, respektive des erzeugten Aerosols, Schaden nehmen könnte.

Die WO 01/64268 beschreibt eine weiteres Gerät dieser Art: einen nadellosen Injektor, der mit einem dem Gerät der WO 97/12687 ähnlichen Druckbeaufschlagungsmittel arbeitet.

Ein weiteres Gerät, welches nicht auf der zuvor genannten Technologie aufbaut, wird in der EP 0918570 beschrieben. Hier wird ein Zerstäuber für Nasensprays offenbart, der als Kernelemente einen federbetriebenen Kolben und eine Düseneinrichtung enthält. Zwischen Kolben und Düse kann ein Behälter eingelegt werden, der bodenseitig einen Stempel aufweist und kopfseitig über eine Dichtung verschlossen ist. Diese Dichtung des Behälters wird vor dem ersten Gebrauch durch Bewegen der in dem Zerstäuber integrierten Düse geöffnet, indem die Düse durch die Dichtung hindurch geschoben wird.

Die beschriebenen Geräte aus dem Stand der Technik sind primär für den kontinuierlichen Gebrauch bestimmt, d.h. für einen Gebrauch ohne längere Unterbrechungen. Bei einer längeren zeitlichen Unterbrechung kann der Teil des Lösungsmittels der flüssigen Wirkstoffformulierung, der sich außerhalb des Reservoirs in nur kleinen Volumina in dem Pump- und/oder Druck- und/oder Sprühmechanismus befindet, verdunsten und dort zu einer Formulierung mit aufkonzentrierter Menge an Wirkstoff führen oder die Formulierung trocknet ein. In diesen Fällen, muss das Gerät vor Wiedergebrauch zunächst durch ein oder mehrfache Betätigung und Versprühens der Wirkstoffformulierung in die Luft wieder gereinigt werden.

### Beschreibung der Erfindung

Die vorliegende Erfindung betrifft nun ein Gerät, welches aufbauend auf der Respimat^{®}-Technologie, die Aufgabe hat, eine diskontinuierliche, also gelegentliche Verabreichung einer flüssigen Medikamentenformulierung mit reproduzierbarer Dosiergenauigkeit bereit zu stellen.

Eine weitere Aufgabe besteht darin, in solchen Fällen auf Reinigungsschritte zwischen den diskontinuierlichen Applikationen verzichten zu können.

Eine weitere Aufgabe besteht darin, einen Vernebler für die diskontinuierliche Verabreichung von flüssigen Wirkstoff-Formulierungen bereit zu stellen, in denen ein die pharmazeutische Qualität der Formulierung oder die pharmazeutische Qualität der Applikation gefährdendes Eintrocknen von Flüssigkeit im System weitgehend minimiert wird.

Eine weitere Aufgabe besteht darin, ein solches Gerät bereit zu stellen, bei dem auf den Einsatz von Konservierungsstoffen in Wirkstoff-Formulierungen gegebenenfalls verzichtet werden kann.

Eine weitere Aufgabe besteht darin, ein solches Gerät bereit zu stellen, mit dem auch flüssige Wirkstoff-Formulierungen vernebelt werden können, die unter Normalbedingungen (d.h. unter Luft oder Sauerstoffatmosphäre) oder bei nicht steriler Handhabung schnell an pharmazeutischer Qualität einbüßen.

Schließlich ist es die Aufgabe der vorliegenden Erfindung, eine Vorrichtung zur Abgabe einer dosierten Menge einer flüssigen Arzneistoffformulierung als ein Flüssigkeitsstrahl oder als Aerosol aus Tröpfchen durch Abgabe einer dosierten Menge des Arzneistoffs unter Druck durch Ausbringungseinrichtung zur Verfügung zu stellen, welche die vorerwähnten Nachteile der bekannten Vorrichtungen nicht aufweist.

Eine weitere Aufgabe der Erfindung besteht darin, einen Vernebler zur Bereitstellung eines inhalierbaren Aerosols bereitzustellen.

Eine weitere Aufgabe der Erfindung besteht darin, einen nadellosen Injektor zur Bereitstellung eines sich selbst in oder durch die Haut eines Menschen/Tieres oder eine menschliche, tierische oder pflanzliche Membran injizierenden Strahls bereitzustellen.

Eine weitere Aufgabe der Erfindung besteht darin, einen Zerstäuber zum Aufbringen eines Aerosols auf die Augenoberfläche bereitzustellen.

Eine weitere Aufgabe der Erfindung besteht darin, ein Gerät zum Ausbringen von pharmazeutischen Flüssigkeiten zur nadellosen Injektion, Inhalation, Vernebelung etc. bereit zu stellen, welches den hohen hygienischen Anforderungen eines Medizingeräts genügt.

### Detaillierte Beschreibung der Erfindung

Entgegen den bekannten Vorrichtungen, die als Multidosis-Geräte so konzipiert sind, daß in der Regel ein Gerät die gesamte zur Ausbringung der Flüssigkeit vorgesehene Technik enthält und dieses Gerät mit einem Arzneistoffbehälter bestückt ist, der so viel an Arzneistoff enthält, daß dem Patienten bis zu mehreren hundert Einzeldosen verabreicht werden können, liegt der Erfindung ein völlig anderes erfinderisches Konzept zugrunde.

Erfindungsgemäß wird eine Vorrichtung bereitgestellt, bei der die zur Ausbringung der Flüssigkeit erforderliche Technik in folgende Teilaspekte zerlegt wird: Die zur Ausbringung der Flüssigkeit notwendige Technik und die dafür notwendigen Bauelemente auf wenigstens zwei konstruktiv getrennte Bauteile aufgeteilt. Zum einen einem Bauteil (Primärpackmittel), welches die zur Bevorratung des Arzneimittels notwendigen Elemente und die mit dem Arzneimittel in unmittelbare Berührung kommenden Elemente stellt. Zum anderen einem zweiten Bauteil, das die Elemente enthält, welche die Energie und die Mechanik für den Ausbringungsprozess bereit stellen.
Erfindungsgemäß wird das Primärpackmittel nach seiner Einführung in das zweite Bauteil mittels eines Transportmittels in seine Endposition überführt.

Demnach wird damit zum einen eine erfindungsgemäße Vorrichtung zum Ausbringen einer Flüssigkeit geschaffen, und zum anderen ein Vorratsbehälter zur Aufnahme der Flüssigkeit mit einer daran integrierten oder mit diesem Behälter fest verbundenen Ausbringungseinrichtung als integralem Bestandteil. Bevorzugt wird dieser Behälter als Behälterpatrone geschaffen, die in die Vorrichtung zum Ausbringen der Flüssigkeit eingesetzt wird.

Die Vorrichtung zum Ausbringen der Flüssigkeit enthält dabei
a) Mittel zum Einführen und Herausnehmen der den Arzneistoff enthaltenden Behälterpatrone in bzw. aus dem Inneren der Vorrichtung und
b) Mittel zum Ausüben von Druck auf die Behälterpatrone.
Diese Vorrichtung wird im Rahmen der vorliegenden Erfindung auch Vorrichtung zur Druckbeaufschlagung oder Gerät genannt.
Diese Vorrichtung ist wiederverwendbar, d.h. sie ist auf eine Vielzahl von Einzelbetätigungen ausgelegt und dient im wesentlichen dazu, die Behälterpatrone samt Ausbringungseinrichtung aufzunehmen und die in dem Behälter befindliche Flüssigkeit über die Ausbringungseinrichtung des Behälters auszubringen. Dafür stellt die Vorrichtung einen Mechanismus zum Beaufschlagen von Druck auf den Behälter bzw. der Flüssigkeit in seinem Inneren zur Verfügung.

Die erfindungsgemäße Behälterpatrone selbst enthält neben der integrierten Ausbringungseinrichtung Mittel, die den durch die Vorrichtung erzeugten Druck auf die Flüssigkeit im Inneren des Behälters weitergeben, um die so mit Druck beaufschlagte Flüssigkeit der Ausbringungseinrichtung zuzuführen.

Der Behälter wird im Rahmen dieser Erfindungsbeschreibung auch Vorratsbehälter, Behälterpatrone oder nur Patrone genannt.

Die Behälterpatrone enthält das Arzneimittel und dient damit als Primärpackmittel. Zusätzlich enthält der Behälter all jene Elemente, die mit dem Arzneimittel direkt in Berührung kommen. Dazu zählt insbesondere die eigentliche Ausbringungseinrichtung, die bevorzugt eine Düse ist. Der Behälter kann beispielsweise als wegwerfbarer Behälter, z.B. als Einwegbehälter ausgebildet sein.

Wie bereits ausgeführt, handelt es sich bei der Flüssigkeit bevorzugt um eine pharmazeutische Formulierung, z.B. Arzneimittel-Lösungen oder Arzneimittel-Suspensionen.

Durch die erfindungsgemäße Bereitstellung eines druckerzeugenden Geräts und eines davon unabhängigen Primärpackmittels für die Arzneistoffzubereitung werden die der Erfindung zugrunde liegenden Aufgaben erfüllt. Da mit jeder Applikation eine neue Behälterpatrone in der Vorrichtung zur Bereitstellung der Druckbeaufschlagung verwendet werden kann, wird hierdurch beispielsweise gewährleistet, daß selbst wenn die Vorrichtung über einen längeren Zeitraum nicht verwendet wird, die Arzneimittelformulierung keinen Schaden nimmt, da die Patrone noch unbenutzt ist.

Die Tatsache, daß die Behälterpatronen dergestalt sein können, daß sie nur eine einzige Dosiseinheit aufnehmen, d.h. nur eine einzige Applikation möglich ist bzw. die Menge und Arzneimittel nur für wenige Dosierungen ausreicht, erlaubt es, konservierungsmittelfreie Arzneimittel einzusetzen. Dies hat nicht nur eine geringe Belastung des Patienten mit antimikrobiell wirksamen Substanzen zur Folge, sondern ermöglicht auch Arzneimittelformulierungen von Arzneistoffen (wie z. B. Peptide), die nicht stabil zusammen mit zur Inhalation zugelassenen Konservierungsstoffen formuliert werden können. Die mit der erfindungsgemäßen Vorrichtung realisierbare Konservierungsmittelfreiheit gestattet daher, Arzneistoffe, die aufgrund der Unverträglichkeit mit Konservierungsmitteln bislang nicht formulierbar waren, überhaupt verfügbar zu machen.

### Beschreibung der Vorrichtung zur Druckbeaufschlagung

Wie eingangs geschildert baut der wiederverwertbare Teil der Erfindung auf der Respimat^{®}-Technologie auf. Dieser Teil umfasst eine Vorrichtung mit a) Mittel zum Einführen und Herausnehmen der den Arzneistoff enthaltenden Behälterpatrone in bzw. aus dem Inneren der Vorrichtung und b) Mittel zum Ausüben von Druck auf die Behälterpatrone. Dabei baut diese Vorrichtung auf dem Prinzip auf, wie es durch die WO 97/12687 und deren Figuren 6 beschrieben wird.

Bevorzugt ist diese Vorrichtung von Zylinder-ähnlicher Form und weist eine handliche Größe von weniger als 9 bis 15 cm in der Länge und 2 bis 4 cm in der Breite auf, so daß sie vom Patienten jederzeit mitgeführt werden kann.

Sie weist ein bodenseitiges Ende und gegenüber liegend ein kopfseitiges Ende auf. Das kopfseitige Ende definiert die Richtung "oben", das bodenseitige Ende die Richtung "unten". Das Kopfteil weist am oberen Ende eine Öffnung auf, durch die die auszubringende Flüssigkeit aus dem Gerät austritt.

Bevorzugt besteht die Vorrichtung aus wenigstens drei Gehäuseabschnitten, a) einem bodenseitigen Gehäuseunterabschnitt, b) einem Gehäusemittelabschnitt und c) einem kopfseitigen Gehäuseoberabschnitt.

Sofern die beiden Abschnitte Gehäusemittelabschnitt und Gehäuseoberabschnitt eine konstruktive Einheit bilden oder im Kontext nicht zwischen den beiden Abschnitten differenziert werden muss, werden beide Abschnitte als oberer Gehäuseabschnitt zusammengefasst.

Der nach oben hin offene Gehäuseoberabschnitt kann durch einen Deckel oder eine Klappe verschlossen werden. Dieser Deckel/Kappe kann integraler Bestandteil des selben sein oder ein davon separiertes Element darstellen.
Der Gehäuseoberabschnitt ist bevorzugt drehbar oder klappbar mit dem Gehäusemittelabschnitt verbunden.

Der Gehäuseunterabschnitt kann auf den Gehäusemittelabschnitt in axialer Richtung aufgesteckt oder mit ihm verbunden werden.

Bevorzugt beinhaltet der Gehäusemittelabschnitt eine Feder, die über eine Drehbewegung des Gehäuseunterabschnitts gegen den Gehäusemittelabschnitt gespannt wird.

Der Gehäuseoberabschnitt dient zur Aufnahme des Behälters und weist entsprechende Mittel auf.
Der Gehäuseoberabschnitt weist parallel zur Längsachse des Geräts (= vertikale Richtung) eine durchgehende, bevorzugt röhrenförmige, d.h. zylindrische Bohrung auf. Dadurch wird ein gegebenenfalls zylinderartiger Hohlraum ausgebildet, der nach zwei Seiten hin offen ist. Dieser Hohlraum ist zur Aufnahme der Behälterpatrone ausgebildet und wird im Rahmen dieser Beschreibung auch Behälteraufnahmekammer oder kurz Aufnahmekammer genannt. Alternativ wird sie als Gehäuseöffnung bezeichnet.

Die Behälterpatrone kann manuell oder über ein Transportmittel in die Aufnahmekammer hinein- und herausgeschoben werden. Die Aufnahmekammer ist dabei bevorzugt dergestalt daß die Behälterpatrone passgenau aufgenommen wird. D.h. die Behälterpatrone soll im Innenraum selbst keine oder annähernd keine Querbewegung ausführen können. Handelt es sich bei der Behälterpatrone zum Beispiel um eine flaschenähnlichen Behälterpatrone, d.h. einem Behälter mit Bauch-, Schulter- und Kopfbereich, ist demnach der Innenraum der Aufnahmekammer bevorzugt komplementär dazu ausgebildet, d.h. diese Flaschenform wird als negative Form nachgebildet. Bei allen Ausführungsformen der Erfindung muss gewährleistet sein, daß der Behälter wenigstens kurzzeitig fest mit der Aufnahmekammer verbunden ist, damit der Behälter bei Druckbeaufschlagung nicht aus der Aufnahmekammer herausgeschleudert wird.

Die beiden Öffnungen der Aufnahmekammer liegen einander gegenüber, wobei eine zum Boden des Geräts zeigt und im geschlossenen Zustand des Geräts die Decke des Gehäusemittelabschnitts berührt. Die andere Öffnung weist zum Kopfteil und mündet bevorzugt in einen nach oben hin ebenfalls offenen, geraden und rohrförmigen Vorsprung, der auf der Kopfseite des Gehäuseoberabschnitts ausgebildet ist und dessen Höhenachse bevorzugt ebenfalls parallel zur Längsachse des Geräts ausgebildet ist. D.h. die auf der Öffnungsebene des Rohrs stehende Senkrechte liegt parallel zur Längsachse des Geräts. Dieser Vorsprung kann ein Mundstück für einen Inhalator, ein Adapter für eine Augendusche oder dergleichen sein, oder eine solche Einrichtung kann mit dem Vorsprung verbunden werden. Ein solcher Adapter für eine Augendusche wird in der PCT/EP0207038 beschrieben, auf die hiermit ausdrücklich Bezug genommen wird. Ein Mundstück wird beispielsweise in den Figuren 6 a/b der WO 97/12687 beschrieben, auf die hiermit auch in diesem Zusammenhang ausdrücklich verwiesen wird.

Durch die kopfseitige Öffnung der Aufnahmekammer kann ein aus der Behälterpatrone austretendes Aerosol durch den rohrförmigen Vorsprung hindurch aus dem Gerät austreten. Bevorzugt passt die Behälterpatrone genau in die Aufnahmekammer.

In einer Ausführungsform kann in dem oberen Gehäuseabschnitt oder an dem oberen Gehäuseabschnitt ein Transportmittel ausgebildet oder befestigt sein, insbesondere ein Schlitten bzw. ein Transportschlitten, in das die Behälterpatrone eingelegt wird und das dann die Behälterpatrone in ihre Endposition in der Aufnahmekammer überführt.

In einer weiteren Ausführung der Erfindung kann vorgesehen sein, daß ein Teil der oberen Gehäusewand Bestandteil eines entfernbaren Griffstücks ist, welches mit einer Halterung zur Aufnahme des Behälters vorgesehen ist. Durch das Entfernen eines Teils der Gehäusewand entsteht auf diese Weise eine Öffnung, durch die der Behälter in das Innere der Vorrichtung eingebracht werden kann. Dieser herausnehmbare Teil der Gehäusewand ist mit einer geeigneten Halterung versehen, mit welcher eine exakte Positionierung des Behälters in seine Sollposition besonders leicht und schnell möglich ist.

In anderen Ausführungsformen, bei denen der Gehäusemittelabschnitt und der Gehäuseoberabschnitt ebenfalls untrennbar miteinander verbunden sein können und so eine konstruktive Einheit darstellen, kann die Behälterpatrone nur von oben, vom kopfseitigen Ende der Aufnahmekammer in diese eingebracht werden. Auch in diesem Fall ist es so, daß das bodenseitige Ende der Behälterpatrone zum bodenseitigen Ende der Aufnahmekammer weist. Über ein Gewinde kann die Behälterpatrone fest mit der Aufnahmekammer verbunden sein, damit sie während der Druckbeaufschlagung nicht herausgeschleudert werden kann. In diesem Fall trägt dann die Behälterpatrone z.B. ein Außengewinde und die Aufnahmekammer ein komplementäres Innengewinde. Ein solcher Verschluß kann auch als BajonettVerschluß ausgebildet sein, oder an der Äufnahmekammer sind entsprechende Halterungen ausgebildet.

In weiteren Ausführungsformen, die kein solches Transportmittel für den Behälter aufweisen oder bei denen die Behälterpatrone nicht von oben, sondern nur von unten in die Aufnahmekammer eingebracht werden kann, kann der Gehäuseoberabschnitt zumindest partiell lösbar mit dem Gehäusemittelabschnitt verbunden sein. In einem solchen Falls sind die beiden Abschnitte auf jeden Fall so miteinander verbunden, daß der Gehäuseoberabschnitt so vom Gehäusemittelabschnitt entfernt werden kann, daß die bodenseitige Öffnung der Aufnahmekammer zugänglich ist. Gleichzeitig weist das Gerät Verschlußmittel auf, die dafür sorgen, daß dieser Öffnungsmechanismus nur bewusst vom Nutzer des Geräts ausgeführt werden kann und ein zufälliges Trennen des Gehäuseoberabschnitts vom Gehäusemittelabschnitt während der Benutzung des Geräts nicht möglich ist.

In einer solche Ausführungsform kann vorgesehen sein, daß zur Einbringung des Behälters der Gehäuseoberabschnitt um den Gehäusemittelabschnitt exzentrisch drehbar oder klappbar geschwenkt wird. Durch die schwenkbare Ausführung unter Zuhilfenahme z. B. eines Scharniers oder Drehgelenks öffnet sich die Gesamtvorrichtung und das Innere der Vorrichtung wird zugänglich. In diesem Zustand kann die Behälterpatrone in die bodenseitige Öffnung der Aufnahmekammer im Innenbereich der Vorrichtung eingebracht werden. Der Vorteil dieser Ausführungsform besteht darin, daß das Scharnier oder das Drehgelenk die Funktionsweise des Mechanismus gut visualisiert und das Öffnen somit selbsterklärend ist. Aufgrund der klaren Bedienzonen wird ein sich in der Aufnahmekammer befindlicher Eindosisbehälter nach dem Öffnen sofort sichtbar und die Art und Weise, wie die Behälterpatrone ausgetauscht werden muss, ist offensichtlich.

In all diesen Fällen mit einem gegenüber dem Gehäusemittelabschnitt beweglichen Gehäuseoberabschnitt kann die bodenseitige Öffnung der Aufnahmekammer für den Behälter in der Auflagefläche von Gehäuseoberabschnitt und Gehäusemittelabschnitt liegen bzw. den Gehäusemittelabschnitt berühren oder fast berühren.

Der Öffnungsmechanismus kann dergestalt sein, daß der Gehäuseoberabschnitt an der Außenseite über ein exzentrisches Drehgelenk mit dem Gehäusemittelabschnitt verbunden ist. Dadurch wird eine Drehbewegung des Gehäuseoberabschnitts in der durch die Längsachse des Geräts definierten Querebene möglich, d.h. einer horizontale Drehbewegung, bei der die Achse zwischen bodenseitigem Ende des Gehäuseoberabschnitts und seinem kopfseitigen Ende stets parallel oder annähernd parallel zur Längsachse des Geräts ausgerichtet bleibt.

Es kann auch vorgesehen sein, daß der Gehäuseoberabschnitt als Schwenkklappe ausgebildet ist. In einem solchen Fall, kann der Gehäuseoberabschnitt vom Gehäusemittelabschnitt weggeklappt werden, d.h. die Achse zwischen bodenseitigem Ende des Gehäuseoberabschnitts wird so bewegt, als würde sie auf den Kopf gestellt. Die Schwenkklappe umfasst hierbei z.B. ein Scharnier, das bevorzugt am unteren Ende des Gehäuseoberabschnitt angeordnet ist.

Bei Ausführungsformen, bei denen der Gehäuseoberabschnitt zunächst geöffnet werden muss, bevor die Behälterpatrone eingesetzt werden kann, ist es vorteilhaft, wenn das Gehäuseoberteil nicht vollständig vom Gehäusemittelabschnitt getrennt werden kann. Das erleichtert die Bedienbarkeit des Geräts.

Der Hohlraum der Aufnahmekammer ist in derartigen Ausführungsformen derart, daß darin der Behälter nur von unten in die Aufnahmekammer eingeschoben werden kann. In diesem Fall ist der Kopfbereich der Behälterpatrone in Richtung der kopfseitigen Öffnung ausgerichtet und der Bodenbereich der Behälterpatrone zeigt in Richtung des Gehäuseunterabschnitts. Im Kopfbereich des Behälters liegt die Ausbringungseinrichtung welche ggf. eine Zerstäubungseinrichtung, im Idealfall eine Düse darstellt. Diese kann dabei noch innerhalb der Behälteraufnahmekammer liegen, mit der Aufnahmekammer abschließen oder durch die kopfseitige Öffnung hinaus ragen. Der analoge Fall gilt für das bodenseitige Ende der Behälterpatrone. Bevorzugt schließt der Boden der Behälterpatrone plan mit der bodenseitigen Öffnung der Aufnahmekammer ab.

Die Aufnahmekammer und der Behälter sind bevorzugt derart gestaltet, daß der Behälter nur von unten nicht jedoch von oben in die Aufnahmekammer eingeschoben werden kann. Gegebenenfalls sind am Behälter und/oder der Aufnahmekammer weitere Mittel ausgebildet, die verhindern, daß der Behälter ganz durch die Aufnahmekammer hindurch geschoben werden kann. Diese Mittel können aus Führungsschienen, Führungsrillen oder Führungseinkerbungen entlang der vertikalen Achse der Kammer bestehen, aus Anschlägen und ähnlichem. Der Behälter weist dann dazu konträre Mittel auf. Beispielsweise kann die bodenseitige Öffnung der Aufnahmekammer z.B. im Anfangsbereich eine oder mehrere Aussparungen aufweisen und der Behälter weist bodenseitig entsprechende Vorsprünge auf, die in die Aussparungen passen. Auch kann an der bodenseitigen Öffnung der Aufnahmekammer z.B. eine Aussparung in Form eines umlaufenden Rings (Kragen) ausgebildet sein. Im Längsschnitt hat damit der Hohlraum der Aufnahmekammer eine T-förmige Gestalt. Der Behälter kann dann genau komplementär dazu ausgebildet sein, also im Längsschnitt ebenfalls T-förmig, wobei der "T-Balken" den Boden des Behälters bildet. In diesem Fall kann der Behälter bodenseitig einen Ring oder Kragen aufweisen, der den Außenmantel so verdickt, daß er in den Bereich der Aussparung passt, aber nicht mehr in den Bereich mit dem kleineren Querschnitt der Aufnahmekammer.

In anderen Ausführungsformen verjüngen sich der Behälter und die Aufnahmekammer zum oberen Ende.
Auch kann am kopfseitigen Ende der Aufnahmekammer ein Anschlag ausgebildet sein, der dafür sorgt, daß z.B. der Behälter nicht gänzlich durch diese Öffnung hindurch geschoben werden kann. Der Anschlag, z.B. in Form einer sich verjüngenden Öffnung oder einer nach innen ausgebildeten umlaufenden Kante, kann so ausgebildet sein, das das Kopfende des Behälters oder im Fall eines flaschenförmigen Behälters dessen Schulter an dem Anschlag anstößt. Da erfindungsgemäß die Ausbringungseinrichtung bevorzugt das Kopfende der Behälterpatrone bildet, d.h. im Fall einer flaschenähnlichen Behälterpatrone der Flaschenhals, kann ein solcher Anschlag dazu führen, daß die Ausbringungseinrichtung von dem Anschlag gehalten wird oder aber die Behälterpatrone wird unterhalb der Ausbringungseinrichtung im Schulterbereich gehalten und die Ausbringungseinrichtung selbst ragt durch die Öffnung in den röhrenförmigen Vorsprung.

Bevorzugt sind Ausführungsformen, bei denen der Behälter vollständig nur von unten in die Ausführungskammer eingeschoben werden kann. Bei Ausführungsformen, bei denen der Behälter zumindest ein geringes Stück von der oberen Öffnung kommend in die Aufnahmekammer hinein geschoben werden kann, sind am Behälter und / oder der Aufnahmekammer Sperrelemente ausgebildet, die ein vollständiges Einschieben des Behälters verhindern. Hierbei muss darauf geachtet werden, daß der Behälter nicht soweit in die Öffnung hinein geschoben werden kann, daß von der Druckaufschlagvorrichtung Druck auf den Behälter übertragen werden kann.

Eine bevorzugte Ausführungsform weist für den Gehäuseoberabschnitt als weiteres Bauelement eine schwenkbare und arretierbare Schutzkappe auf, die zumindest den röhrenförmigen Vorsprung und damit die kopfseitige Öffnung der Aufnahmekammer oder den oberen Deckenbereich bedeckt. Damit wird gewährleistet, daß die weiter innenliegenden Bereiche der Vorrichtung geschützt werden. Dies ist insbesondere wichtig, wenn das Gerät in der Hosentasche oder einer Handtasche aufbewahrt wird. Damit die Schutzkappe nicht selbst ungewollt aus ihrer arretierten Stellung gelangt, kann vorgesehen sein, daß die Schutzkappe über einen zungenförmigen Abschnitt verfügt, welcher in einer zungenförmigen Ausnehmung des Gehäuses einrastbar ist. Diese Schutzkappe kann so ausgebildet sein, daß sie im geschlossenen Zustand den Auslöseknopf des Geräts, welcher im Gehäusemittelabschnitt ausgebildet ist, überdeckt und dadurch ein ungewolltes Auslösen verhindert.

Der Gehäusemittelabschnitt beherbergt einen Energiespeicher zur Erzeugung von Druck auf den Behälter und ein bewegliches Element, das durch Freisetzen der gespeicherten Energie bewegt wird und dadurch direkt oder indirekt Druck auf die Behälterpatrone ausübt bzw. auf die in seinem Inneren befindliche Flüssigkeit.
Bevorzugt handelt es sich bei dem Energiespeicher um ein elastisches Element, beispielweise eine Druckfeder (Springfeder). Die Druckbeaufschlagung kann aber auch mittels anderer Elemente beispielsweise eines Motors bewerkstelligt werden.
Im Fall einer Druckfeder oder Springfeder als Energiespeicher kann diese in einem Druckfedergehäuse angeordnet sein, welches sich zumindest teilweise in dem Gehäusemittelabschnitt befindet und gegebenenfalls mit diesem über Schnappverschlüsse verbunden ist. Bevorzugt ragt zumindest ein Teil des Druckfedergehäuses bodenseitig aus dem Gehäusemittelabschnitt heraus, d.h. das Druckfedergehäuse ist länger als der Gehäusemittelabschnitt. In diesem Fall kann das Druckfedergehäuse oder ein Teil davon mittels eines Drehlagers drehbar gelagert sein, um über eine Drehbewegung und ein Sperrspannwerk die Druckfeder zu spannen. Durch einen Auslösemechanismus kann die Druckfeder dann wieder entspannt werden.
Das bewegliche Element kann ein Kolben (Druckkolben) sein, der durch die Druckfederbewegung selbst bewegt wird. Dabei wird er durch die Entspannung der Druckfeder in die Aufnahmekammer geschoben und übt dabei Druck auf den Behälter aus. Der Druckkolben kann über einen Abtriebsflansch, mit der Druckfeder in Verbindung stehen, wobei er in diesem Fall fest mit dem Abtriebsflansch verbunden ist. Der Druckkolben wird bevorzugt über eine Bohrung im ansonsten verschlossenen Deckenbereich des Gehäusemittelabschnitts geführt. Ggf. kann der kopfseitige Teil des Druckkolbens dabei in einem zylinderförmigen Element (Führungszylinder) geführt werden, welches im Deckenbereich des Gehäusemittelabschnitts ausgebildet ist. Im gespannten Zustand der Druckfeder befindet sich der Druckkolben vollständig im Gehäusemittelabschnitt. Im entspannten Zustand befindet sich das obere Ende des Druckkolbens im Gehäuseoberabschnitt und dringt dabei in eine dort befindliche Behälterpatrone ein. Der Druckkolben hat dabei einen vertikalen Bewegungsspielraum von bis zu einigen Zentimetern, bevorzugt weniger als 2 cm, besonders bevorzugt zwischen 0,1 und 1,5 cm.
Der Druckkolben kann als Hohl- oder Vollkolben ausgebildet sein und übt nach Aktivierung hohen mechanischen Druck auf den Behälter aus

Das Sperrspannwerk enthält die besagte Druckfeder, bevorzugt eine zylindrische schraubenförmige Druckfeder, als Speicher für die mechanische Energie. Das Sperrspannwerk weist bevorzugt eine vertikale Längsachse auf. Im folgenden wird eine Ausführungsform des Sperrspannwerks beschrieben. Die Druckfeder wirkt auf einen Abtriebsflansch als Sprungstück, dessen Bewegung durch die Position eines Sperrglieds bestimmt wird. Der Weg des Abtriebsflansches wird durch einen oberen und einen unteren Anschlag präzise begrenzt. Die Druckfeder wird bevorzugt über ein kraftübersetzendes Getriebe, z.B. ein Schraubschubgetriebe, durch ein äußeres Drehmoment gespannt, das beim Drehen des Gehäusemittelabschnitts gegen das Druckfedergehäuse im Gehäuseunterabschnitt erzeugt wird. In diesem Fall enthalten der Gehäusemittelabschnitt und der Abtriebsflansch ein Keilgetriebe, welches ein- oder mehrgängig ist.
Der Abtriebsflansch wird gegen die Kraft der Druckfeder in das Druckfedergehäuse gepresst.

Über ein Sperrglied kann die Druckfeder im gespannten Zustand gehalten werden.
Dieses Sperrglied weist einrückende Sperrflächen auf und ist ringförmig um den Abtriebsflansch angeordnet. Es besteht z.B. aus einem Ring aus Kunststoff oder aus Metall. Der Ring ist in einer Ebene senkrecht zur Zerstäuberachse angeordnet und ist in dieser Ebene beweglich gelagert. Nach dem Spannen der Druckfeder schieben sich die Sperrflächen des Sperrgliedes in den Weg des Abtriebsflansches und verhindern das Entspannen der Druckfeder. Das Sperrglied wird mittels einer Taste ausgelöst (Auslösetaste), die ebenfalls am Gehäusemittelabschnitt ausgebildet ist. Dieser Auslassvorgang kann durch Drücken der Taste bewirkt werden. Die Auslösetaste ist mit dem Sperrglied verbunden oder gekoppelt. Zum Auslösen wird die Auslösetaste parallel zur Ringebene, und zwar bevorzugt in den Zerstäuber hinein, verschoben; dabei wird sich der Ring in der Ringebene bewegen. Konstruktive Details des Sperrspannwerkes sind in der WO 97/20590 beschrieben, bezüglich des Sperrmechanismus wird auf die Figuren 3 folgend dieser Patentanmeldung verwiesen. Alternativ dazu kann der Ring radial elastisch verformbar sein. In diesem Fall wird der Ring verformt, wenn die Auslösetaste zum Auslösen bewegt wird. Eine Bewegung des Ringes in der Ringebene ist dabei nicht notwendig.

Am Gehäuseoberabschnitt und/oder Gehäusemittelabschnitt können Mittel ausgebildet sein, welche die beiden Abschnitte so miteinander verbinden, daß ein Trennen, Aufklappen etc. der beiden Abschnitte während der Druckauslösung nicht möglich ist.

In solchen Fällen ist der Gehäusemittelabschnitt mit dem Gehäuseoberabschnitt über einen Verschluß verbunden, der verhindert, daß sich der Gehäuseoberabschnitt nicht unbeabsichtigt öffnet.
Zu diesem Zweck kann der Gehäusemittelabschnitt Mittel zum Blockieren (Blockiermittel) des Auslösemechanismus aufweisen, die Verhindern, daß die Druckbeaufschlagung ausgelöst werden kann, wenn das Gerät geöffnet ist, d.h. solange der Gehäuseoberabschnitt nicht fest mit dem Gehäusemittelabschnitt verbunden ist.

Außerdem kann der Gehäusemittelabschnitt Mittel aufweisen, die Verhindern, daß das Gerät geöffnet werden kann (der Gehäuseoberabschnitt geöffnet werden kann), solange die Druckfeder entspannt ist, und damit der Kolben den Gehäuseoberabschnitt hineinragt (Verschlußarretiermittel).

Eine bevorzugte Ausführungsform weist sowohl Blockiermittel auf als auch Verschlußarretiermittel auf.

Bevorzugt sind die Blockiermittel derart, daß sie die Bewegung des Sperrgliedes (s. Sperrspannwerk) in die Richtung verhindern, in die das Sperrglied gedrückt wird, um die Druckfeder zu entspannen. Ein solches Mittel kann ein federgelagerter Sperrbolzen sein, der aus horizontaler Ebene vom Druckknopf aus gesehen vertikal hinter dem Sperrglied liegt. Im geöffneten Zustand eines z.B. kippbaren Gehäuseoberabschnitts drückt eine Feder den Sperrbolzen etwas nach oben aus dem Gehäusemittelteil heraus. Im geschlossenen Zustand drückt der Gehäuseoberabschnitt den Sperrbolzen gegen die Feder zurück in die Ausgangsposition. Der Sperrbolzen kann zylindrisch, quadratisch und dergleichen sein und ist entweder so gestaltet oder so geführt, daß der Sperrbolzen die zum Auslösen notwendige Horizontalbewegung des Sperrglieds verhindert wenn das Gerät geöffnet ist und das Sperrglied frei gibt, wenn das Gerät geschlossen ist. Z.B. kann der Sperrbolzen Aussparungen aufweisen, die nur im geschlossenen Zustand des Geräts den Weg des Sperrglieds zum Entspannen der Druckfeder freigeben.

Der Sperrbolzen kann in Alternativausführungen auch die Auslösetaste so arretieren, daß diese nur eingedrückt werden kann, wenn das Gerät geschlossen ist. In einer solchen Ausführungsform kann der Sperrbolzen wiederum Aussparungen aufweisen. Auch in diesem Fall steht dann der Sperrbolzen einer Bewegung der Auslösetaste so lange im Weg, bis der Sperrbolzen gedrückt ist und dadurch den Weg der Auslösetaste freigibt.

Analoge Blockiermittel können für Ausführungsformen des Geräts ausgebildet sein, bei denen der Gehäuseoberabschnitt exzentrisch drehbar gegenüber den Gehäusemittelabschnitt gelagert ist.
In diesen Fällen wird der Sperrbolzen über die Drehbewegung zurück in seine Ausgangsposition gedrückt, und gibt so den Weg für das Sperrglied oder die Auslösetaste frei.

Das Verschlußarretiermittel ist bevorzugt mit dem Verschluß zwischen Gehäuseoberabschnitt und Gehäusemittelabschnitt gekoppelt. Es verhindert, daß der Gehäuseoberabschnitt geöffnet werden kann, solange der Druckkolben in den Gehäuseoberabschnitt hineinragt, also die Druckfeder entspannt ist. Es kontrolliert dann die Freigabe der Taste, die den Verschluß zwischen den beiden Gehäuseabschnitten hält (Verschlußtaste).

Die Verschlußtaste ist dann mit einem Arretierbolzen gekoppelt. Dieser steht mit dem Druckkolben in mechanischer Verbindung. Z.B. kann er horizontal oder zumindest windschief zur Längsachse des Druckkolbens gelagert sein. Ist die Druckfeder gespannt, befindet sich der Druckkolben innerhalb des Gehäusemittelteils. Der Arretierbolzen kann dann durch eine Feder oberhalb des Druckkolbens in dessen Führungskanal geschoben werden. In dieser Position wird die Verschlußtaste frei gegeben, so daß der Verschlußmechanismus zwischen Gehäusemittelabschnitt und Gehäuseoberabschnitt geöffnet werden kann.

In Alternativausführungen schiebt sich der Arretierbolzen nicht in den Führungskanal des Druckkolbens, sondern entlang einer Aussparung des Druckkolbens. Die Aussparungen am Druckkolben geben nur im gespannten Zustand der Druckfeder für den Arretierbolzen den Weg frei. Im entspannten Zustand der Druckfeder steht dagegen der Druckkolben dem Arretierbolzen im Weg, so daß die Freigabebewegung des Arretierbolzens solange verhindert wird, solange der Druckkolben nicht gänzlich in das Gehäusemittelabschnitt versenkt ist. Wird das Gerät entspannt, bewegt sich der Druckkolben von unten nach oben und schiebt dabei den Arretierbolzen in seine Ausgangslage zurück, in der er die Verschlußtaste blockiert. Zu diesem Zweck können die Spitze des Druckkolbens oder die Aussparung Schrägen aufweisen und der Arretierbolzen weist die entsprechenden komplementären Schrägen auf.

Der Gehäuseunterabschnitt befindet sich unterhalb des Gehäusemittelabschnitts. Er wird in bevorzugten Ausführungsformen axial über das Druckfedergehäuse geschoben bis sich der Gehäuseunterabschnitt und der Gehäusemittelabschnitt berühren, während das Druckfedergehäuse sich innerhalb des dadurch gebildeten Raums befindet.
Der Gehäuseunterabschnitt wird dabei über eine lösbare Verbindung, z.B. eine Steckverbindung oder unlösbare Verbindung mit dem Druckfedergehäuse verbunden.

Beim Betätigen des Geräts wird der Gehäusemittelabschnitt gegen den Gehäuseunterabschnitt gedreht, wobei der Gehäuseunterabschnitt das Druckfedergehäuse mitnimmt. Dabei wird die Druckfeder über das Schraubschubgetriebe zusammengedrückt und gespannt, und das Sperrwerk rastet selbsttätig ein. Der Drehwinkel ist bevorzugt ein ganz-zahliger Bruchteil von 360 Grad, z.B. 180 Grad. Gleichzeitig mit dem Spannen der Druckfeder wird das Abtriebsteil im Gehäusemittelabschnitt um einen vorgegebenen Weg verschoben und der Druckkolben geführt vom Zylinder im Deckenbereich des Gehäusemittelabschnitts wird zurückgezogen. Weitere konstruktive Details sind in den PCT-Anmeldungen WO 97/12683 und WO 97/20590 offenbart, auf die hiermit inhaltlich Bezug genommen wird.

### Beschreibung der Behälterpatrone

Bei der Behälterpatrone handelt es sich um einen formstabilen Behälter, der durch manuellen Druck nicht verformt werden kann, d.h. er ist weder entlang der Längsachse noch seiner Querachse plastisch verformbar. Bevorzugt ist der Kolben so ausgelegt, daß er formstabil ist gegenüber einem Druckunterschied von Innen nach Außen von 49 bis 599 bar, bevorzug von 149 bis 299 bar.

Wie bereits ausgeführt ist der Behälter bzw. die Eindosiskartusche als wegwerfbarer Teil fest mit einer Einrichtung zum Ausbringen einer Flüssigkeit, beispielsweise mit einer Düse, verbunden. D.h. diese Einrichtung ist integraler Bestandteil des Behälters. Damit benötigt die Vorrichtung zur Druckbeaufschlagung (das Gerät) keine eigene Ausbringungseinrichtung mehr, so daß diese Vorrichtung zur Druckbeaufschlagung als solches gegenüber den aus dem Stand der Technik bekannten Geräten der Marke Respimat® baulich vereinfacht wird.

Die Behälterpatrone ist bevorzugt von zylinderähnlicher oder flaschenähnlicher Gestalt. Die Gestalt des Behälters kann auch patronenförmig sein oder der Gestalt einer Inhalationskapsel nachgebildet sein. Die äußere Gestalt des Behälters muss dabei kein getreues Abbild eines Zylinders, einer Flasche, Patrone oder Inhalationskapsel sein, bevorzugte Ausführungsformen ähneln jedoch einem der Gegenstände. Die Gestalt einer Inhalationskapsel kann den Figuren der EP 1100474 entnommen werden, auf die hiermit Bezug genommen wird. Solche Kapseln können als zylinderähnliche Gebilde mit zwei halbkreisförmigen Enden beschrieben werden. Die Behälterpatrone weist ein bodenseitiges und ein kopfseitiges Ende auf, wobei das bodenseitige Ende zum bodenseitigen Ende der Vorrichtung zur Druckbeaufschlagung zeigt, wenn der Behälter in dieses Gerät eingesetzt ist. Entsprechend weist das kopfseitige Ende der Behälterpatrone zum kopfseitigen Ende der Vorrichtung zur Druckbeaufschlagung.

In einer bevorzugten Ausführungsformen ist die Behälterpatrone von rotationssymmetrischer Außenkontur, die sich von unten nach oben verjüngt. Dabei ist bevorzugt, daß der Durchmesser sich in Stufen verjüngt. Am stärksten bevorzugt ist wenigstens eine derartige Stufe ausgebildet, so daß der Behälter eine Schulter aufweist. Eine solcher Behälter kann z.B. von flaschenähnlicher Gestalt sein, mit Fußteil, Bauchteil, Schulter und Kopfteil. Bevorzugt weist der Fußbereich einen peripher um die Ummantelung laufenden Kragen auf oder ist im Bodenbereich verbreitert. Dies kann über eine entsprechende Bodenplatte erreicht werden. Diese Form hat den Vorteil, daß sich der Behälter nur in richtiger Orientierung und nur von der unteren Öffnung die die zum Behälter passgenaue Aufnahmekammer des Druckgeräts schieben lässt.

In alternativen Ausführungsformen ist der zur Längsachse vertikale Querschnitt des Behälters dreieckig, viereckig, mehreckig oder weist eine andere nicht rotationssymmetrische Form auf. Der Vorteil dieser Formen besteht darin, daß der Behälter vom Benutzer sehr bewusst in die Aufnahmekammer geschoben werden muss, so daß Fehler vermieden werden. In einer solchen Ausführungsform kann der Querschnitt z.B. die Form eines Kreissektors haben, also eines Gebildes mit drei Ecken, zwei geraden und einer gekrümmten Seite. Der Winkel zwischen den beiden geraden Seiten kann dabei beliebige Werte zwischen mehr als 0 Grad und weniger als 360 Grad annehmen, bevorzugt sind Werte von 200 Grad bis 300 Grad.

Alternativ dazu kann der Querschnitt die Form eines Kreissegements sein, also einem Gebilde das durch eine Gerade und einem die Gerade überspannendem Bogen aufweist, bzw. ein Kreis, bei dem parallel zum Durchmesser ein Stück abgeschnitten ist. Die Höhe über der Mittelsenkrechten der Gerade kann dabei größer als der Radius des zugrunde liegenden Kreises sein, gleich groß oder kleiner. Bevorzugt ist die Höhe größer als der Radius. Auch bei diesen Ausführungsformen ist eine Verjüngung von unten nach oben, ggf. stufenartig, vorteilhaft.

In wieder alternativen Ausführungsformen ist bei den zuvor beschriebenen Behältern die gegenüber dem Rest des Behälters breitere Bodenplatte des Behälters selbst von kreisrundem Querschnitt.

In weiteren Ausführungsformen ist der Querschnitt des Behälters rund, während die gegenüber dem Rest des Behälters breitere Bodenplatte (Fuß) des Behälters die zuvor beschriebenen nicht rotationssymmetrischen Querschnitt aufweist.

Bevorzugt handelt es sich bei der Behälterpatrone um einen Ein-Dosis-Behälter oder eine Ein-Dosis-Kartusche. Dieser Behälter weist einen hohlen Zylinder zur Aufnahme des Fluids auf (Vorratszylinder), die eigentliche Vorratskammer, die im Gebrauch auch als Druckkammer fungiert. In dem Vorratszylinder kann sich bodenseitig ein verschiebbar angeordnetes Element (verschiebbarer Behälterstempel, z. B. in Form eines Kolbens (Behälterkolben) oder bevorzugt einer Kugel (Behälterkugel)) befinden, welches das Fluid nach außen abdichtet. Am kopfseitigen Ende des Behälters ist die Einrichtung zum Ausbringen der Flüssigkeit angeordnet. Dabei sind der verschiebbar angeordnete Behälterstempel, die Vorratskammer und die Ausbringungseinrichtung so in Reihe angeordnet, daß eine Flüssigkeit, die sich in dem Vorratszylinder, d.h. in der Vorratskammer, befindet durch die Ausbringungseinrichtung gepresst wird, wenn der Behälterstempel durch eine von Außen einwirkende Kraft in die Vorratskammer hinein gedrückt wird. Bei Gebrauch mit dem Gerät zur Druckbeaufschlagung handelt es sich bei der von Außen einwirkenden Kraft um die Kraft, die durch den Druckkolben auf den Behälterstempel ausgeübt wird. Im Fall von einer Arzneistofflösung bzw. -suspension als bevorratete Flüssigkeit wird diese der Zerstäubungseinrichtung zugeführt. Diese ist bevorzugt eine Zerstäuberdüse, welche ihrerseits zur Vernebelung des Arzneistoffes führt.

Gegebenenfalls können die bodenseitige Öffnung und die kopfseitige Öffnung des Vorratszylinders eine Versiegelung oder mehrere Versiegelungen aufweisen.

Die Versiegelung der bodenseitigen Öffnung kann dabei entweder bodenseitig vom Behälterstempel angeordnet sein oder in kopfseitiger Richtung. Bevorzugt dichtet der Behälterstempel selbst die bodenseitige Öffnung ab. Ggf. wird eine Versiegelungsfolie bodenseitig auf die bodenseitige Öffnung aufgebracht.

Die Versiegelung des kopfseitigen Endes kann ebenfalls in bodenseitiger Richtung, d.h. vor der Ausbringungseinrichtung angeordnet sein oder danach, also kopfseitig. Bevorzugt ist sie kopfseitig angeordnet, d.h. die Öffnung oder die Öffnungen der Ausbringungseinrichtung ist (sind) versiegelt, z.B. durch eine manuell ablösbare Siegelfolie.

Bevorzugt wird vorgesehen, daß der Vorratszylinder innerhalb des Behälters zur Bevorratung ein Volumen von maximal 1 ml aufweist, bevorzugt sind Volumina von maximal 100 Mikrolitern, z.B. für die Augenbehandlung, besonders bevorzugt sind Volumina von weniger als 50 Mikrolitern. Für die nasale Anwendung können Volumina von bis zu 30 Mikrolitern bevorzugt sein und für die pulmonal-inhalative Anwendung sind am stärksten bevorzugt Volumina von bis zu 15 -20 Mikrolitern. Diese Arzneistoffmenge ist ausreichend für die Verabreichung einer einzelnen Dosis und vermeidet wunschgemäß den Einsatz eines Konservierungsmittels.

In einer bevorzugten Ausführungsform weist der Vorratszylinder über die gesamte Längsachse einen gleichbleibenden Innendurchmesser auf. Die bodenseitigen und kopfseitigen Öffnungen befinden sich senkrecht zur Längsachse an der Oberseite bzw. der Unterseite des Vorratszylinders. Beide Öffnungen erstrecken sich über den gesamten Durchmesser des Vorratszylinders.

Der Behälter weist bevorzugt eine Höhe von bis zu 4 cm, stärker bevorzugt von bis zu 2,5 cm auf, besonders bevorzugt bis zu 2 cm. Der Vorratszylinder in seinem Inneren weist ein entsprechende Länge auf und entsprechend ist das Verhältnis von Länge zu Querschnitt, um das gesamte Füllvolumen bereit zu stellen. Der Durchmesser des Querschnitts beträgt bevorzugt bis zu 5 mm, stärker bevorzugt bis zu 3 mm und besonders bevorzugt bis zu 2,5 mm.

Der Behälterstempel liegt passgenau in dem Vorratszylinder und ist bevorzugt aus einem Kunststoff gefertigt. Dieser kann beispielsweise sein: Polytetrafluorethylen, Ethylen-Propylen-Dienpolymer, Silikon, Elastomere, thermoplastische Elastomere, wie Santoprene® und andere.

Bevorzugt liegt der Behälterstempel ausschließlich innerhalb des Vorratszylinders und stärker bevorzugt schließt das bodenseitige Ende des Behälterkolbens bodenseitig mit dem Behälter ab, d.h. der Behälterstempel ragt nach Außen nicht über den Boden des Behälters hinaus und kann daher auch nicht zufällig bei der Lagerung, Transport und dergleichen bewegt werden.

Der Behälterstempel ist passgenau oder annähernd passgenau dimensioniert, so daß er den Vorratszylinder zum einen dicht verschließt, zum anderen jedoch unter Aufwendung einer Kraft in den Vorratszylinder hinein bewegt werden kann.
Unter dem Begriff passgenau oder annähernd passgenau wird verstanden, daß der Behälterstempel den Vorratszylinder dem Querschnitt nach ausfüllt, gegebenenfalls kann der bezüglich des Verschlusses des Vorratszylinders verantwortliche Durchmesser des Behälterkolbens um bis zu 5% breiter sein als der Durchmesser des Vorraszylinders. Unter annähernd passgenau wird verstanden, daß dieser Durchmesser des Behälterstempels geringfügig kleiner ist als der Durchmesser des Vorratszylinders.

Bevorzugt ist der Behälterstempel als passgenauer Behälterstempel ausgebildet. Eine solche Variante kann beim Befüllen des Vorratszylinders von Vorteil sein, aber auch beim Durchführen des Behälterstempels durch den Vorratzylinder.

Der Behälterstempel kann einen geringfügig größeren Außendurchmesser als der Innendurchmesser des Vorratszylinders aufweisen, insbesondere wenn er sich in Verschlußstellung innerhalb des Vorratszylinders befindet. Dadurch wird ein besserer Verschluß der bodenseitigen Öffnung erreicht. Zusätzlich hat das den Vorteil, daß der Behälterstempel den Vorraszylinder vollständig entleert, wenn der Behälterstempel durch den Vorraszylinder hindurch geschoben wird.

In einer Ausführungsform ist der Behälterstempel ein Zylinder.

Ein zylinderförmiger Behälterstempel kann eine Aussparung in Form eines Hohlraums aufweisen, der nach einer Seite hin offen ist. Die Öffnung der Aussparung weist zur bodenseitigen Öffnung des Vorratszylinders, also in Richtung des Druckkolbens. Der Innendurchmesser der Öffnung bzw. der Aussparung ist größer als der Außendurchmesser des Druckkolbens der Vorrichtung zur Druckbeaufschlagung. Im Querschnitt hat der Behälterkolben dann die Form eines U mit ggf. eckig ausgebildeten Kanten. Der Boden der Aussparung bildet die Stelle des Behälterkolbens, an der der Druckkolben angreifen kann, um den Behälterkolben Vorratszylinder zu drücken. Der Vorteil dieser Gestaltung und Anordnung besteht darin, daß sich der Behälterkolben durch den Druck des Druckkolbens auf den Boden der Aussparung an dem gegenüberliegenden Ende leicht verjüngen kann, also an der Seite des Behälterkolbens die Speerspitze beim Durchdringen des Vorratsbehälters bildet. D.h. durch das Drücken des Druckkolbens geht im Querschnitt die Form des Behälterkolbens von der U-Form annäherungsweise in die eines V über. Dadurch wird eine vereinfachte Passage des Behälterkolbens durch Vorratszylinder erreicht. Ein weiterer Vorteil dieser durch den Druck des Druckkolbens verursachten Gestaltänderung des Behälterkolbens besteht darin, den Druck des Druckkolbens auf die den Behälterkolben haltenden Wände zu verringern, so daß selbst ein fest sitzender Behälterkolben gelöst und von dem Druckkolben ohne Verkanten bewegt werden kann.

Um ein Verkanten des Behälterstempels zu verhindern, können an dem Behälterstempel und / oder der Seitenwandung des Vorratszylinders auch Führungseinrichtungen ausgebildet sein, z.B. Führungsschienen oder Führungsrippen etc.
Zur Verbesserung des Gleitens des Behälterstempels durch den Vorratszylinder kann der Behälterstempel oder die Wand des Vorratszylinders mit einem pharmakologisch verträglichen Schmiermittel beschichtet sein. Derartige Schmiermittel sind dem Stand der Technik bekannt und umfassen z.B. Sorbitanester, z.B. Sorbitantrioleat, Ölsäure, Lecithin und andere Fettsäuren, Fettalkohole, Ester von Fettsäuren und dergleichen.

In anderen konstruktiv ähnlich gebauten Behältern, kann der Behälterstempel Teil der steifen und unflexiblen Bodenplatte des Behälters sein. In diesem Fall durchschlägt der Druckkolben die Bodenplatte des Behälters und drückt sich dann in den Vorratszylinder. In solchen Fällen können auf der Bodenplatte Sollbruchstellen ausgebildet sein, so daß der Druckkolben den integrierten Behälterstempel während der Druckbeaufschlagung aus der Bodenplatte leichter herausstechen kann.
In diesen Fällen kann der Druckkolben so dimensioniert sein, daß keine Flüssigkeit aus dem Vorratsbehälter an dem Druckkolben vorbei bodenseitig aus dem Behälter gedrückt wird.

In anderen Ausführungsformen ist die bodenseitige Öffnung des Vorratszylinders nur durch eine flexible Versiegelung, z.B. eine Siegelfolie und dergleichen verschlossen. Bevorzugt ist die Versiegelung nicht unzerstörbar vom Behälter entfernbar. In diesem Fall übernimmt der Druckkolben die Funktion des Behälterstempels.

Die Ausbringungseinrichtung, welche eine Zerstäubungseinrichtung sein kann und die mit dem erfindungsgemäßen Behälter integriert ist, kann eine spezielle Düse sein, wie sie beispielsweise die WO 94/07607, die WO 99/16530 oder die deutsche Patentanmeldung mit der Anmeldenummer 10216101.1 beschreibt. Auf alle Dokumente wird hiermit ausdrücklich Bezug genommen.

Im einfachsten Fall handelt es sich bei der Düse um eine Art Lochblende, d.h. die Düse stellt einen Körper mit einer einzigen zentralen durchgehenden Bohrung dar.

Eine andere Ausführung der Düse ist ein Körper mit wenigstens zwei oder mehr durchgehenden Bohrungen, die parallel zueinander verlaufen oder gegeneinander geneigt sind. Im Fall von gegeneinander geneigten Bohrungen, bildet die Seite mit dem spitzen Winkel die Düsenauslaßseite, die andere Seite entsprechend die Düseneinlaßseite. In Fall von wenigstens zwei Bohrungen beträgt der Neigungswinkel bevorzugt 20 Grad bis 160 Grad, bevorzugt 60 bis 150 Grad, insbesondere bevorzugt 80 bis 100°.
Die Düsenöffnungen sind bevorzugt in einer Entfernung von 10 bis 200 Mikrometern angeordnet, stärker bevorzugt in einer Entfernung von 10 bis 100 Mikrometer, besonders bevorzugt 30 bis 70 Mikrometer. Am stärksten bevorzugt sind 50 Mikrometer.
Die Dimensionen der Düsenöffnungen und Düsenkanäle entsprechen denen, der im Folgenden beschriebenen Ausführungsformen.

Die Düse kann z.B. aus Glas, Silizium, Kunststoff, wie PBT (Polybutadienterephthalat), PP (Polypropylen), PC (Polycarbonat) und anderen bestehen.
Eine andere Ausführungsform der Düse wird in der EP 0860210 beschrieben. Insbesondere wird hiermit ausdrücklich auf die Zeichnungen dieser Patentschrift Bezug genommen. Eine solche Düse besteht aus zwei Teilen, einem Basisteil und einem Deckenteil, die übereinander gelegt werden, um dadurch den eigentlichen Düsenblock zu bilden. Diese beiden Einzelteile können Mikrostrukturen aufweisen, die z.B. durch Ätzen erhältlich sind. Bevorzugt sind die beiden Teile als Platten ausgebildet und die Mikrostrukturen bilden im Inneren des Düsenblocks eine Flüssigkeitsverbindung von der einer Seite zur anderen, nämlich von der Düseneinlaßseite zu der Düsenauslaßseite. Auf der Düsenauslaßseite ist mindestens eine runde oder nicht-runde Öffnung. Bevorzugt hat diese Öffnungen oder im Fall von mehreren haben alle diese Öffnungen eine Tiefe von 2 bis 10 Mikrometer Tiefe und eine Breite von 5 bis 15 Mikrometern, wobei die Tiefe bevorzugt bei 4, 5 bis 6,5 Mikrometern und die Länge bei 7 bis 9 Mikrometern beträgt.
Im Fall von mehreren Düsenöffnungen, bevorzugt sind zwei, können die Strahlrichtungen der Düsen im Düsenkörper parallel zueinander verlaufen oder sie sind in Richtung Düsenöffnung gegeneinander geneigt. Bei einem Düsenkörper mit mindestens zwei Düsenöffnungen auf der Auslaßseite können die Strahlrichtungen - und das ist bevorzugt - gegeneinander geneigt sein, um durch den Aufprall die Flüssigkeit zu zerstäuben.
In diesem Fall beträgt der Neigungswinkel bevorzugt 20 Grad bis 160 Grad, bevorzugt 60 bis 150 Grad, insbesondere bevorzugt 80 bis 100°.
Die Düsenöffnungen sind bevorzugt in einer Entfernung von 10 bis 200 Mikrometern angeordnet, stärker bevorzugt in einer Entfernung von 10 bis 100 Mikrometer, besonders bevorzugt 30 bis 70 Mikrometer. Am stärksten bevorzugt sind 50 Mikrometer.
Die Strahlrichtungen treffen sich dementsprechend in der Umgebung der Düsenöffnungen. Die beiden einzelnen Teile können aus Glas, Silizium oder einem Kunststoff gearbeitet sein. Bevorzugt sind die Mikrostrukturen in eine Siliziumplatte eingeätzt. Beide Teile besitzen wenigsten eine im wesentlichen ebene Oberfläche. Beim Übereinanderlegen der beiden Teile liegen diese beiden Oberflächen aufeinander.
Der Einfachheit halber wird im Folgenden eine Ausführungsform beschrieben, bei der lediglich das Basisteil reliefartige Mikrostrukturen aufweist, nicht jedoch das Deckenteil. In anderen Ausführungsformen ist die Situation gerade umgekehrt oder beide Teile weisen diese Mikrostrukturen auf.
Auf dem Basisteil kann auf der ebenen Oberfläche ein Satz von Kanälen ausgebildet sein, um, im Zusammenwirken mit der im wesentlichen ebenen Oberfläche des Deckenteils eine Vielzahl von Filterdurchgangswegen zu schaffen (Filterkanäle). Daneben kann das Basisteil eine Plenumkammer aufweisen, deren Decke wiederum durch das Deckenteil gebildet wird. Diese Plenumkammer kann den Filterkanälen vor- oder nachgeschaltet sein. Es können auch zwei derartige Plenumkammern ausgebildet sein. Ein anderer Satz von Kanälen auf der im wesentlichen ebenen Oberfläche des Basisteils, der - falls vorhanden - den Filterkanälen nachgeschaltet ist, bildet zusammen mit dem Deckenteil einen Satz von Kanälen, die eine Vielzahl von Düsenauslaßdurchgangswegen schaffen.
Bevorzugt liegt der Gesamtquerschnittsflächenbereich der Düsenauslässe bei 25 bis 500 Quadratmikrometern. Der gesamte Querschnittsflächenbereich beträgt bevorzugt 30 bis 200 Quadratmikrometer.
In einer anderen Ausführungsform weist auch diese Düsenkonstruktion nur eine einzige Düsenöffnung auf.
In anderen Ausführungsformen dieser Art fehlen die Filterkanäle und/oder die Plenumkammer.

Bevorzugt werden die Filterkanäle durch Vorsprünge gebildet, die zick-zackförmig angeordnet sind. So bilden beispielsweise mindestens zwei Reihen der Vorsprünge eine solche zick-zack-Konfiguration. Auch können mehrere Reihe von Vorsprüngen ausgebildet sein, die Vorsprünge jeweils seitlich zueinander versetzt sind, um dadurch zu diesen Reihen windschiefe zweite Reihe aufzubauen, wobei dann diese zuletzt beschriebenen Reihen die zick-zack-Konfiguration bilden. In solchen Ausführungsformen kann der Einlass und der Auslass jeweils einen Längsschlitz für unfiltriertes bzw. filtriertes Fluid aufweisen, wobei jeder der Schlitze im wesentlichen genauso breit ist wie der Filter und im wesentlichen genauso hoch ist wie die Vorsprünge auf den Einlass- bzw. Auslassseiten des Filters. Der Querschnitt der durch die Vorsprünge gebildeten Durchgangspassagen kann jeweils senkrecht zur Strömungsrichtung des Fluids stehen und kann - betrachtet in Strömungsrichtung - von Reihe zu Reihe abnimmt. Auch können die Vorsprünge, die näher zur Einlassseite des Filters angeordnet sind, größer sein als die Vorsprünge, die näher an der Auslassseite des Filters angeordnet sind. Daneben kann sich auch der Abstand zwischen dem Basisteil und dem Deckteil in dem Bereich von der Düseneinlaßseite zur Düsenauslaßseite verjüngen.
Die zick-zack-Konfiguration, die von den wenigstens zwei Reihen von Vorsprüngen gebildet wird, weist einen Neigungswinkel Alpha von bevorzugt 20° bis 250° auf.

Weitere Einzelheiten dieser Düsenkonstruktion können der WO-94/07607 entnommen werden. Auf diese Schrift wird hiermit inhaltlich Bezug genommen, insbesondere auf Figur 1 und deren Beschreibung.

Die beschriebenen Düsen können über einen Düsenhalter mit der Öffnung des Behälters verbunden werden. Ein solcher Düsenhalter ist in der einfachsten Form ein Ring oder Körper mit einer Öffnung, in die die Düse eingesetzt werden kann. Diese Öffnung umfasst den Düsenblock über seien gesamten Mantelfläche, d.h. die Fläche die senkrecht zu der bevorzugt linearen Achse steht, die durch die Düseneinlassseite und die Düsenauslaßseite gebildet wird. Der Halter ist nach oben und unten offen, um weder die Flüssigkeitszufuhr zur Düseneinlassseite der Düse, noch die Ausbringung der Flüssigkeit zu behindern. Dieser Halter kann wiederum in einen zweiten Halter eingesetzt werden. Die äußere Gestalt des ersten Halters ist bevorzugt kegelförmig. Entsprechend ist die Öffnung des zweiten Halters geformt. Der erste Halter kann aus einem Elastomer bestehen.

Die Ausbringungseinrichtung ist mit dem Behälter formschlüssig verbunden und wird hierzu bevorzugt mit dem Behälter über eine Überwurfmutter oder Crimphülse mit jeweils einer offenen Seite verschraubt oder vercrimpt, was besonders preiswert ist. Alternativ kann die formschlüssige Verbindung auch durch Verkleben oder Verschweißen erfolgen, insbesondere mittels Ultraschallschweißens.
In jedem Fall ist die Verbindung derart, daß die Düsenöffnung frei liegt und nicht durch den Verschluß blockiert werden kann.

Im Fall eines nadellosen Injektors ist die Düse derart, daß dadurch ein scharfer Flüssigkeitsstrahl erzeugt wird. Um die Düse herum kann eine trichterförmige Blende (Trichter) ausgebildet sein, deren sich verjüngendes Ende die Düse umgibt. In diesem Fall kann die Düse über die kopfseitige Öffnung der Aufnahmekammer in diese eingebracht werden. Der Trichter ragt dann aus der kopfseitigen Öffnung der Aufnahmekammer heraus. Im Gebrauchsfall wird die breite Öffnung des Trichters auf die Stelle der Haut gesetzt, in welche die Flüssigkeit injiziert werden soll. Durch diese Maßnahme wird ein Verspritzen der Flüssigkeit verhindert.
In anderen Ausführungsformen kann dies Funktion von dem röhrförmigen Vorsprung der Vorrichtung zur Druckbeaufschlagung übernommen werden, wenn dieses entsprechende ausgebildet ist, also der Vorsprung der bei einem Inhalator das Mundstück bildet.

Durch die erfindungsgemäße Vorrichtung zur Druckbeaufschlagung soll in der Behälterpatrone ein Druck erzeugt werden, der das Arzneimittel in dem Behälter mit einem Eingangsdruck von bis zu 600 bar, 50 bar bis 600 bar, besonders bevorzugt 200 bis 300 bar auf dem Düsenkörper presst und es so über die Düsenöffnungen z.B. in ein inhalierbares Aerosol zerstäubt. Die bevorzugten Teilchengrößen eines solchen Aerosols liegen dann bei bis zu 20 Mikrometern, bevorzugt 3 bis 10 Mikrometern. Um diesen Druck aufbauen zu können werden die Dimensionen der Druckkolbenbreite, der Hubhöhe des Druckkolbens, der Durchmesser des Behälterkolbens das Volumen des Vorratszylinders, der jetzt als Druckkammer fungiert und die Kraft der Druckfeder nach den physikalischen Gesetzen entsprechend gewählt.

Neben den eingangs geschilderten Vorteilen der Erfindung kann durch den erfindungsgemäßen Behälters eine höher konzentrierte Nanosuspensionen, d. h. von Suspensionen bei denen die suspendierten Teilchen ca. 100 - 500 nm groß sind, komplikationslos als Strahl ausgebracht oder zerstäubt werden, ohne daß es durch die Einmalverwendung praktisch zu einer Verstopfung der Düsen kommen kann.

Über die erfindungsgemäße Vorrichtung werden bevorzugt Lösungen oder Suspensionen mit jeglicher Art an medizinisch-therapeutisch und/oder medizinisch-prophylaktisch wirksamen Substanzen ausgebracht. Darunter fallen nicht nur niedermolekulare, meist chemischsynthetisch erzeugte, pharmakologisch aktive Substanzen, sondern auch Proteine, Peptide, andere Biomakromoleküle oder Impfstoffe, die in einem solchen Gerät ohne wesentlichen Aktivitätsverlust ausgebracht werden können. Es wird inhaltlich auf die EP 1003478 verwiesen.

Erfindungsgemäß können in die Vorrichtung zur Druckbeaufschlagung gegebenenfalls nacheinander mehrere das auszubringende Fluid enthaltende austauschbare Vorratsbehälter eingeschoben und benutzt werden. Der Vorratsbehälter enthält die entsprechende pharmazeutischen Zubereitungen oder Aerosolzubereitung. In solchen Fällen kann die Vorrichtung zur Druckbeaufschlagung mit einem Revolvermagazin oder einem ähnlichen aus dem bereich der Schnellfeuerpistolen hergeleiteten Magazin bestück sein. Zusätzlich kann dann die Vorrichtung zur Druckbeaufschlagung Mittel aufweisen, die dementsprechend ein automatisches Beladen der Aufnahmekammer mit der Vorratsbehälterpatrone erlauben.

Der Ausbringungsvorgang wird durch leichtes Eindrücken der Auslösetaste eingeleitet. Dabei gibt das Sperrwerk den Weg für das Abtriebsteil frei. Die gespannte Druckfeder schiebt den Druckkolben in den Vorratszylinder des Behälters hinein. Das Fluid tritt aus der Düse des Behälters - ggf. in zerstäubter Form - aus.

Wie bereits geschildert werden einen Inhalator pro Hub bevorzugt Volumina von 10 bis 50 Mikroliter zerstäubt, besonders bevorzugt sind Volumina von 10 bis 20 Mikroliter, ganz besonders bevorzugt ist ein Volumen von 15 Mikroliter.

Alle Bauteile der Druckbeaufschlagungsvorrichtung oder der Behälterpatrone sind aus einem der Funktion entsprechend geeignetem Material. Das Gehäuse und - so weit es die Funktion erlaubt - auch andere Teile sind bevorzugt aus Kunststoff, z.B. im Spritzgießverfahren, hergestellt. Für medizinische Zwecke werden - sofern notwendig - physiologisch unbedenkliche Materialien verwendet.

Bevorzugt wird die Erfindung als Zerstäuber von flüssigen Arzneimittelzubereitungen verwendet.

### Beschreibung der Figuren

Nachfolgend soll die Erfindung anhand von Ausführungsbeispielen näher erläutert werden.
Figur 1 zeigt eine Ausführungsform des erfindungsgemäßes Geräts in zylindersymmetrischer Ausführung.
Figur 2 zeigt ein Gerät inklusive Behälterpatrone.
Figur 3 zeigt eine Ausführungsform mit einem herausnehmbaren Griffstück zur Aufnahme der Behälterpatrone.
Figur 4 zeigt eine Ausführungsform mit gegenüber dem Gehäuseoberabschnitt 2a klappbaren Gehäusemittelabschnitt 2b und Gehäuseunterabschnitt 3.
Figur 5 zeigt eine weitere Ausführungsform des Geräts.
Figuren 6a und 6b zeigen das Öffnen der Schutzkappe des Geräts.
Figur 7 zeigt ein Gerät mit klappbarem Gehäuseoberabschnitt 2a und dessen Beladen mit der Behälterpatrone.

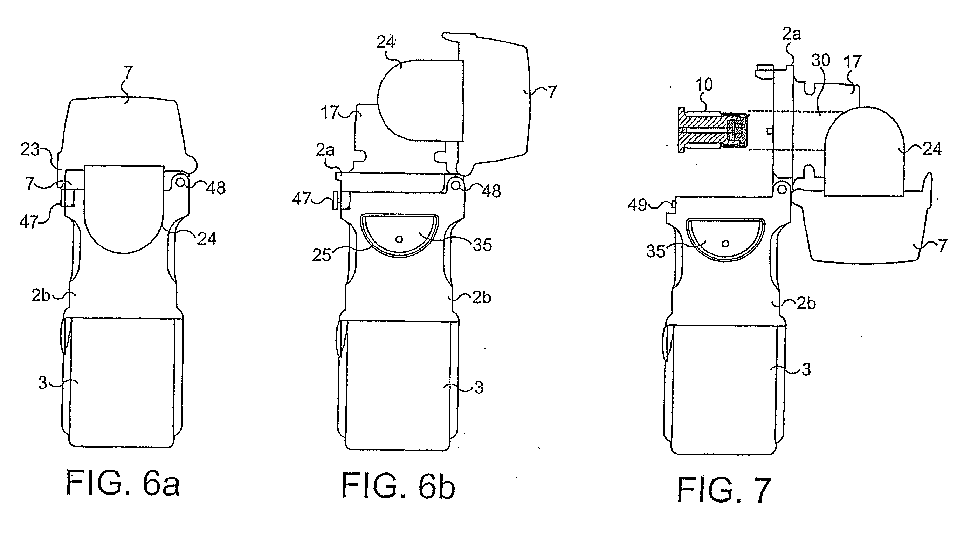

Figur 7 beschreibt schematisch eine Ausführungsform des Geräts.

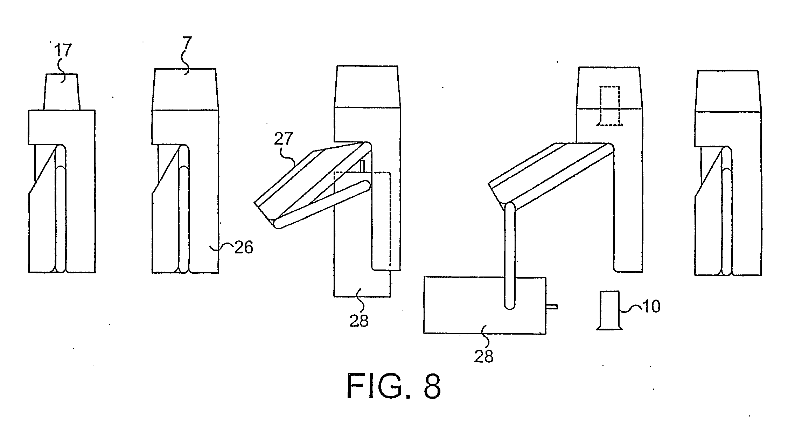

Figur 8 beschreibt schematisch eine weitere Ausführungsform des Geräts mit schwenkbarem Arm.

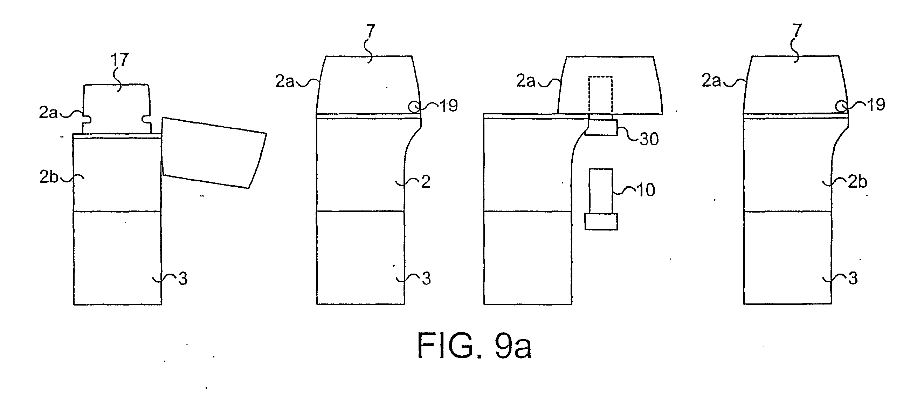

Figur 9a beschreibt schematisch den Vorgang zum Beladen des Geräts mit dem Behälter, wobei der Gehäuseoberabschnitt 2a gegenüber dem Gehäusemittelabschnitt 2b und Gehäuseunterabschnitt 3 horizontal gedreht wird.

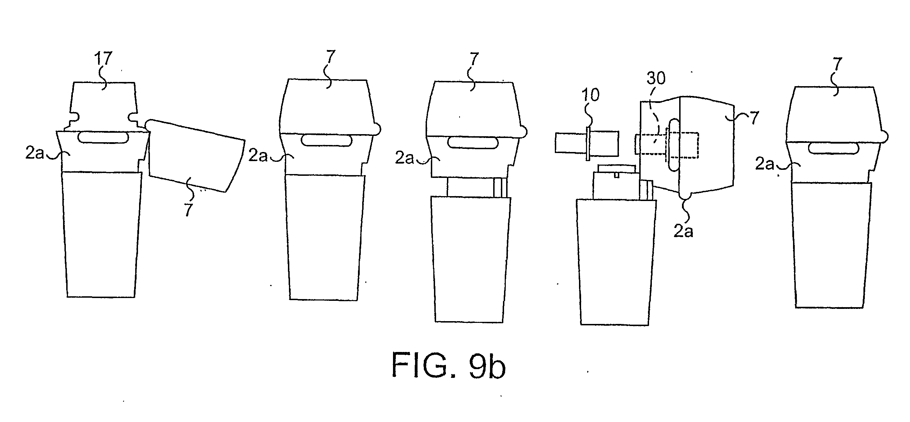

Figur 9b beschreibt schematisch den Vorgang zum Beladen des Geräts mit einem Klappmechanismus mit dem Behälter.

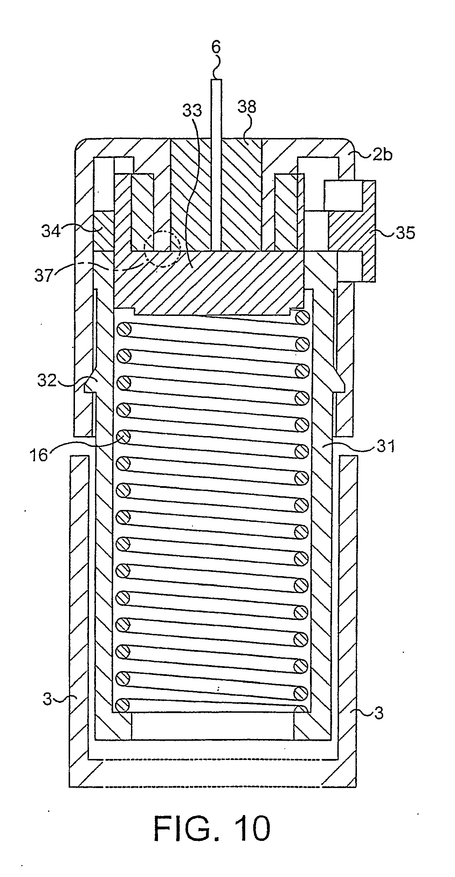

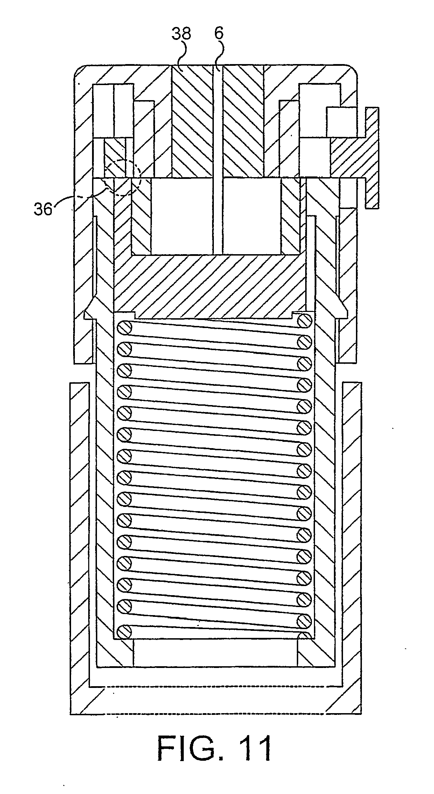

Figur 10 und Figur 11 beschreiben das Druckbeaufschlagungsmittel in Form eines Sperrspannwerks.

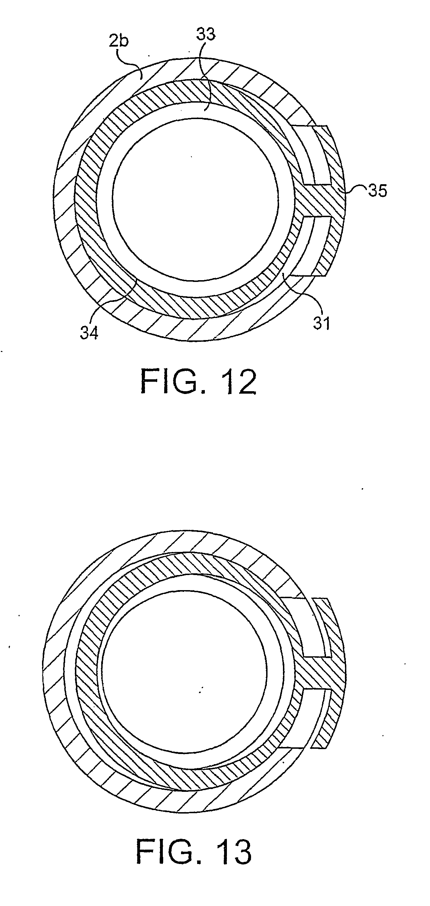

Figuren 12 und 13 zeigen den Sperrmechanismus.

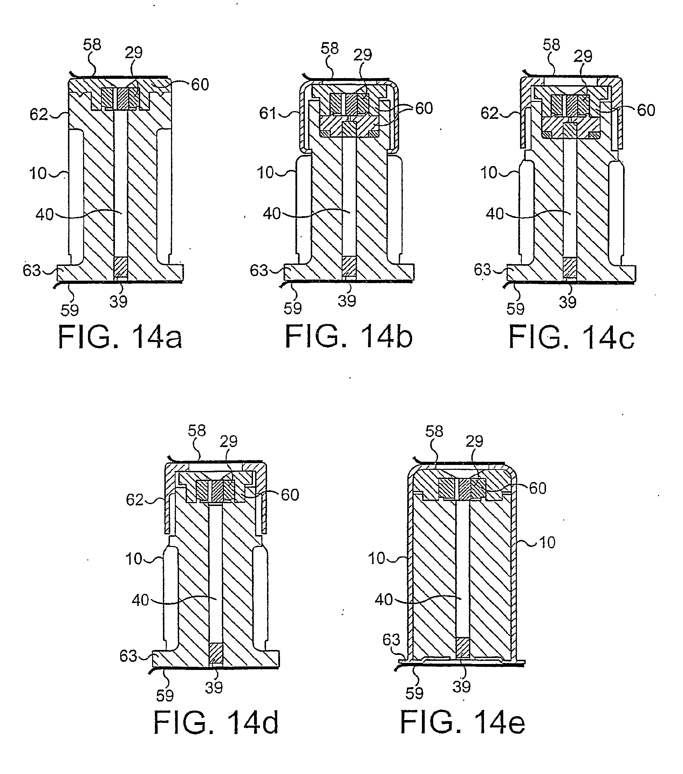

Figuren 14a bis 14e zeigen die erfindungsgemäße Behälterpatrone.

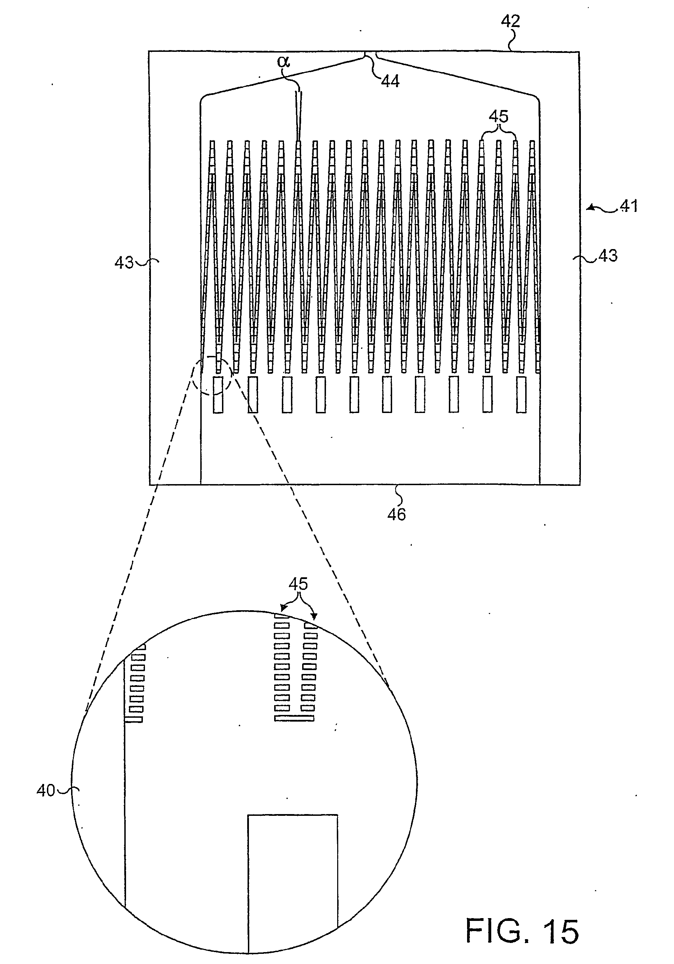

Figur 15 beschreibet eine Ausbringungseinrichtung, bevorzugt zum Zerstäuben einer Flüssigkeit.

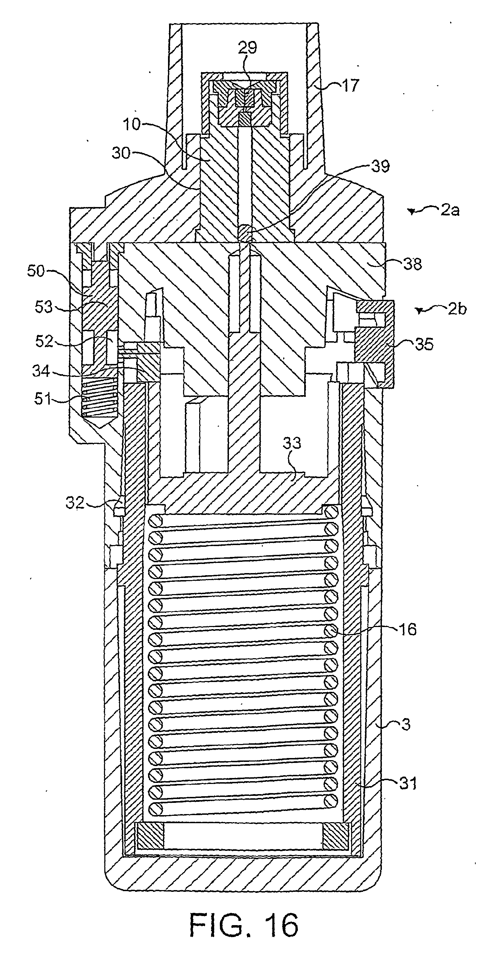

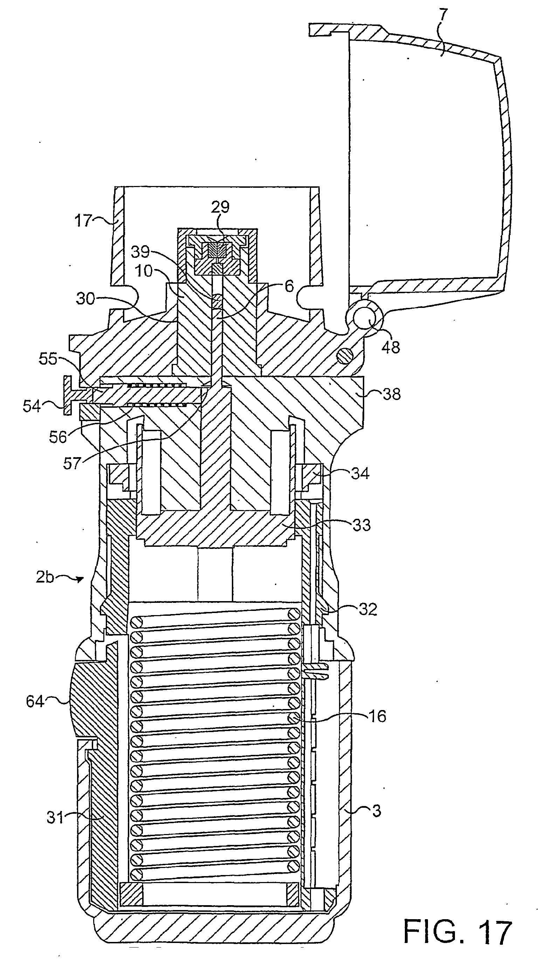

Figur 16 und 17 beschreiben das Gerät im Querschnitt.

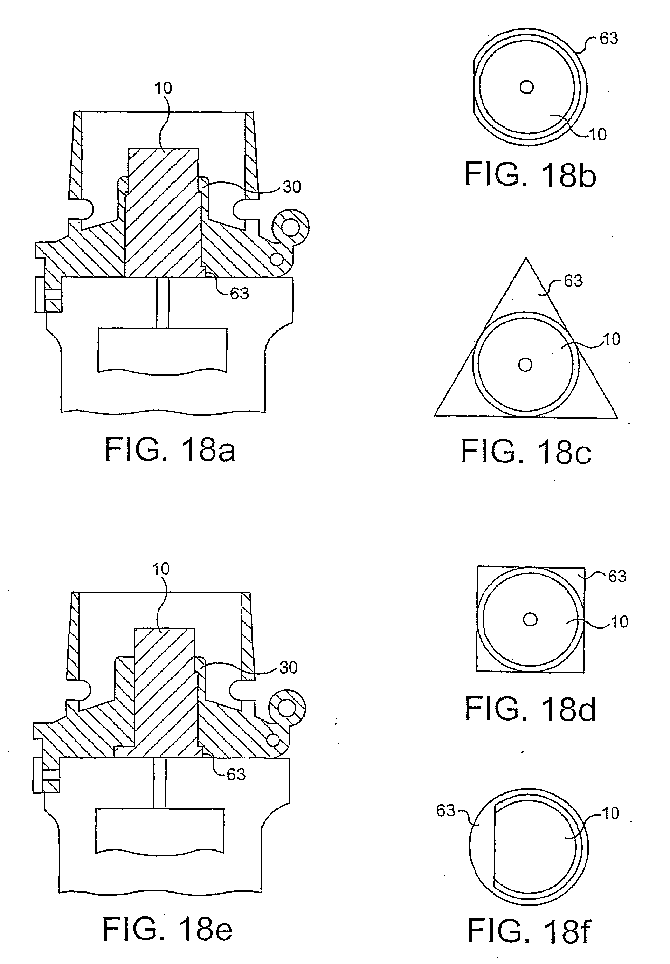

Fig. 18a bis f zeigen verschiedene Behälter mit nicht rotationssymmetrischen Außenkonturelementen.

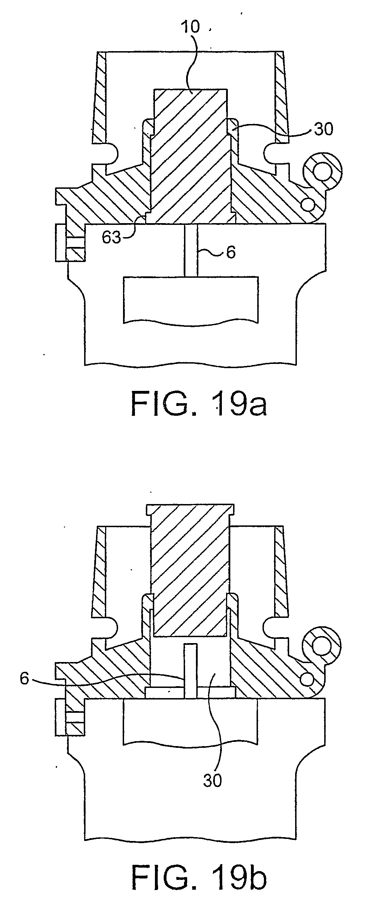

Fig. 19a und 19b zeigen ein Beispiel für eine Aufnahmekammer für einen rotationssymmetrischen Behälter, bei der dieser funktionsgerecht nur von unten in die Aufnahmekammer geschoben werden kann.

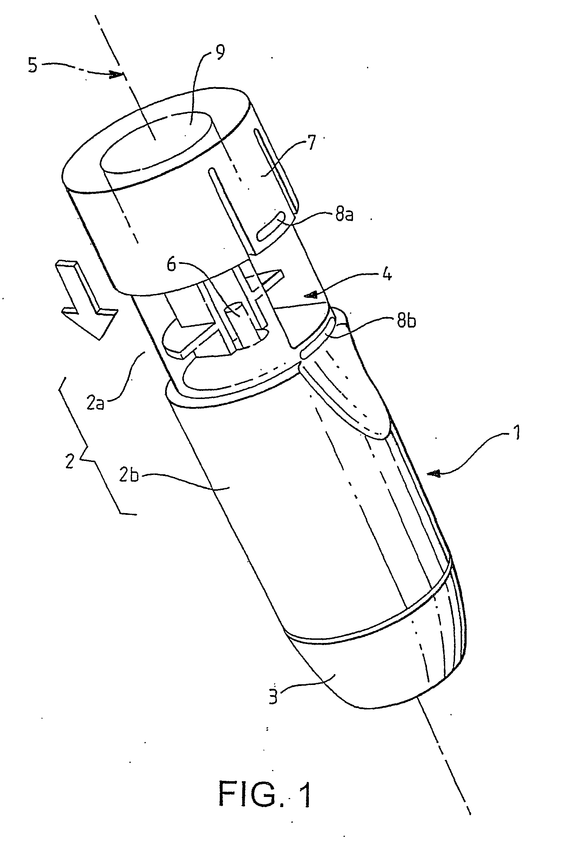

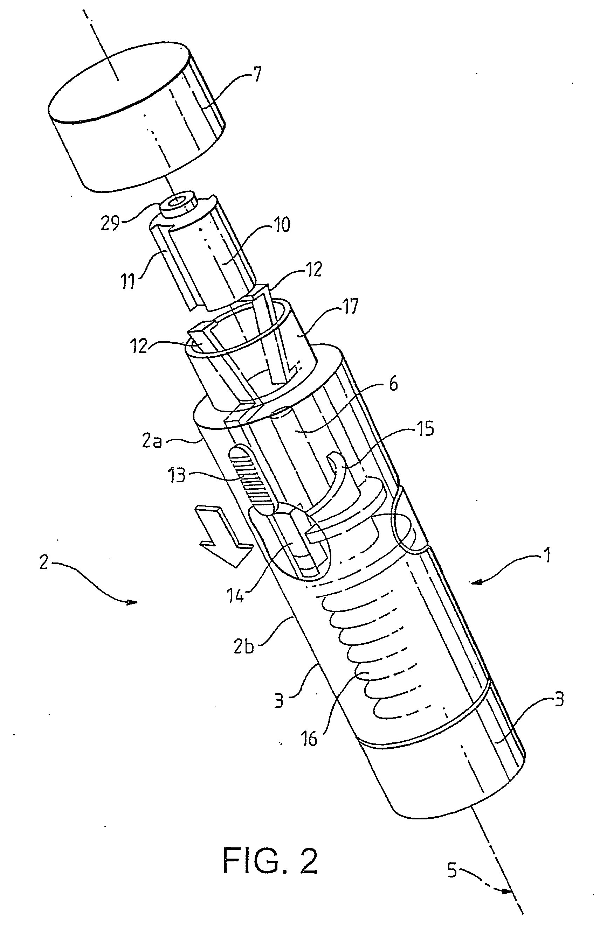

Die Figur 1 zeigt ein erfindungsgemäßes Gerät 1 in zylindersymmetrischer Ausführung. Es besteht aus einem Gehäuseoberabschnitt 2a, dem Gehäusemittelabschnitt 2b, die beide zusammen als konstruktive Einheit auch als 2 bezeichnet werden und Gehäuseunterabschnitt 3. Der obere Gehäuseabschnitt 2 wird von einer Schutzkappe 7 abgedeckt. Das Gerät 1 weist eine Öffnung 4 auf, durch die ein Blick in das Innere ermöglicht ist. Auf der Mitte der Symmetrieachse 5 befindet sich ein bewegliches Element in Form eines Druckkolbens 6 über dem auf direkte Art und Weise die Behälterpatrone 10 (vgl. Fig. 2) liegt. Durch nachfolgende Bewegung der Schutzkappe 7 parallel zur Symmetrieachse 5 in Pfeilrichtung wird das Gerät 1 geschlossen und steht für die Anwendung bereit, bei der z.B. ein Aerosol tropfenförmig aus der Öffnung 9 austritt. Um ein versehentliches Öffnen zu verhindern, ist ein Rastmechanismus 8a, 8b vorgesehen.

Figur 2 zeigt ein ebenfalls zylindrisch ausgeführtes Gerät 1, bei dem nach Entfernen der Schutzkappe 7 die Behälterpatrone 10 von oben in das Innere des Gerätes 1 eingeführt wird.

Hierzu wird die Behälterpatrone 10 entlang der Symmetrieachse 5 in Richtung des Druckkolbens 6 geführt. Die Behälterpatrone 10 weist beidseitig eine Nut 11 auf, die nachfolgend von den Armen der Halterung 12 umfaßt wird. Die Düse bzw. Ausbringungseinrichtung der Behälterpatrone ist als Merkmal 29 gekennzeichnet. Über einen verschiebbaren Knopf 13, welcher in Pfeilrichtung vom Anwender bewegt wird, wird ein Transportschlitten 14 aktiviert, welcher die Behälterpatrone 10 in Richtung des Druckkolbens bewegt. Beim Einsatz des Gerätes sorgen die Druckfeder 16 im Zusammenspiel mit einem Spannelement 15 (Abtriebsflansch) für die schnelle Bewegung des Druckkolbens 6 entlang der Symmetrieachse in Richtung der Behälterpatrone 10. Diese weist einen zeichnerisch nicht dargestellten Vorratszylinder auf, in den der Druckkolben 6 beim Auslösevorgang einsticht und dabei durch Vorschieben eines in die Behälterpatrone 10 eingeordneten Stopfens (Behälterkolben, nicht gezeigt) die im Vorratszylinder befindliche Arzneistofflösung durch die an der Behälterpatrone 10 befindliche Ausbringungseinrichtung (nicht zeichnerisch dargestellt) drückt. Das Aerosol gelangt von der Ausbringungseinrichtung, also in diesem Fall eine Zerstäubungseinrichtung über das Mundstück 17 nach außen.

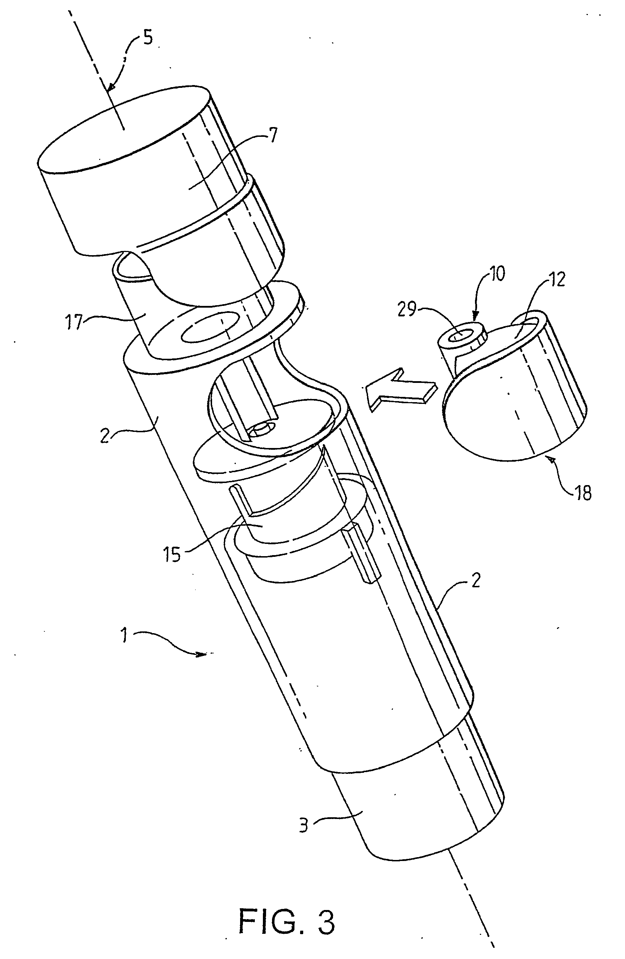

Figur 3 zeigt eine Ausführungsform, bei der ein Teil der Außenwand des Gerätes herausgeschnitten ist, und ein Griffstück 18 bildet, welches mit einer Halterung 12 zur Aufnahme der Behälterpatrone 10 versehen ist. Die Behälterpatrone 10 weist zentrisch eine Ausbringungseinrichtung 29 auf. Nach Bestückung des Griffstücks 18 mit einer neuen Behälterpatrone 10 wird das Griffstück in Pfeilrichtung eingeführt und ist zur Verwendung bereit.

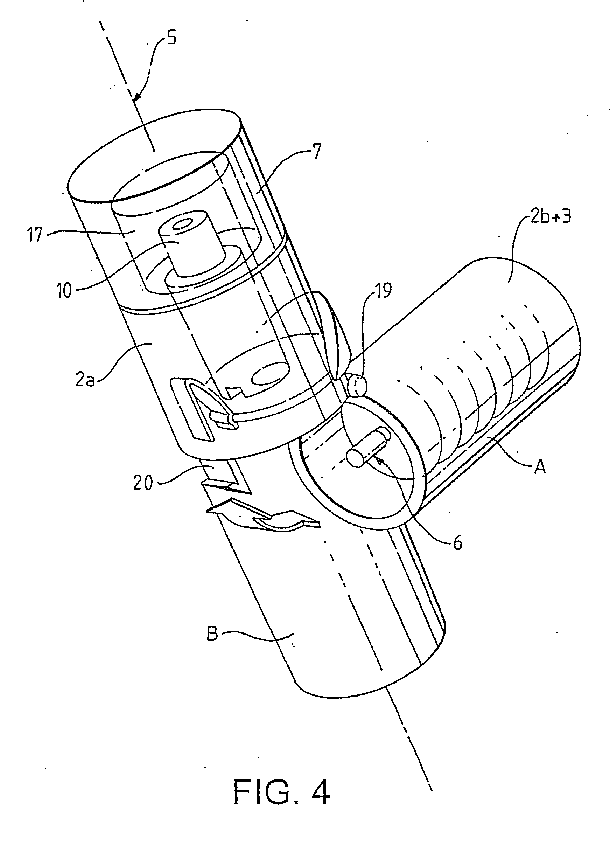

Figur 4 zeigt eine Ausführungsform, bei der der Gehäuseunterabschnitt 3 und der Gehäusemittelabschnitt 2b vereinfacht als Einheit gezeichnet sind. Der Gehäuseoberabschnitt 2a kann gegenüber dem Gehäusemittelabschnitt mittels eines Scharniers 19 verschwenkt werden. Hierzu wird zunächst die Rastnase 20 betätigt, damit der Gehäusemittelabschnitt 2b zusammen mit dem Gehäuseunterabschnitt 3 aus seiner Anfangsposition A in seine Endposition B überführt wird. In ausgeschwenkter Position des Gehäusemittelabschnitts 2b plus Gehäuseunterabschnitt 3 kann der Behälter 10 in den Gehäuseoberabschnitt 2a eingeführt bzw. gewechselt werden. Durch manuellen Druck auf das Kopfende der Behälterpatrone durch das Mundstück 17 kann die Behälterpatrone wieder heraus geschoben werden.

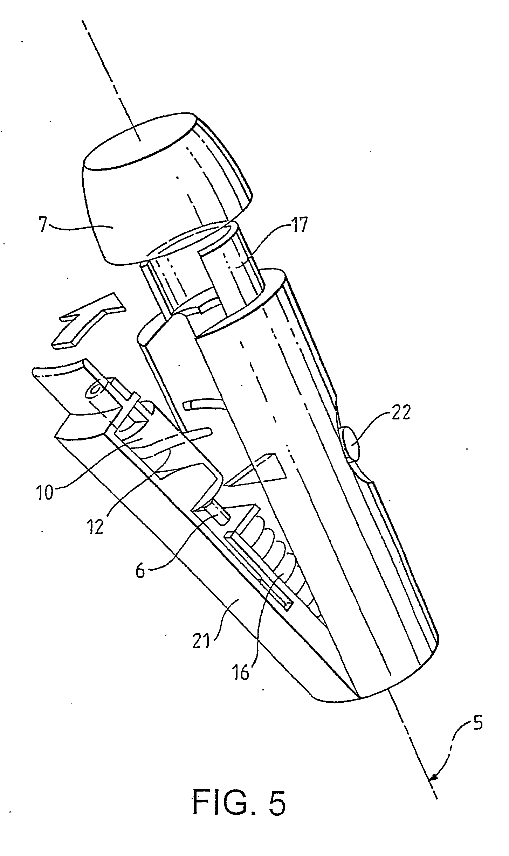

Figur 5 zeigt eine Ausführungsform, bei der ein Teil des Gerätes 1 als Schwenkklappe 21 herausgeschwenkt werden kann. In ausgeschwenkter Position ist ein Blick in das Innere des Gerätes möglich, und man erkennt in Figur 5 eine Drückfeder 16 zum Bewegen des Druckkolbens 6, welcher im Anwendungsfall auf die Behälterpatrone 10 einwirkt, welcher mit einer Halterung 12 in einer Sollposition fixiert wird. Nach Bestückung des Gerätes 1 mit der Behälterpatrone 10 wird die Schwenkklappe 21 in Pfeilrichtung zur Symmetrieachse geklappt. Die Auslösung erfolgt über den Entriegelungsknopf 22.

Die Figuren 6a und 6b zeigen das Gerät mit geschlossener und geöffneter Schutzkappe 7. Der Gehäuseoberabschnitt 2a ist über ein Scharnier 48 mit einer Schutzkappe 7 versehen, welche zunächst nach außen geklappt ist und somit das Mundstück 17 erkennen lässt. Über die Lasche 23 kann die Schutzkappe 7 auf dem Gehäuseoberabschnitt 2a in die Verschlußposition einrasten. Die Schutzkappe 7 weist zudem einen zungenförmigen Abschnitt 24 auf. Dieser Abschnitt 24 bedeckt den zungenförmigen Bereich 25 in dem sich die Auslösetaste 35 befindet. Im geschlossenen Zustand der Schutzkappe 7 kann daher die Auslösetaste 35 nicht ungewollt gedrückt werden.
Am Gehäusemittelabschnitt 2b befindet sich die Verschlußtaste 47. Wird diese betätigt, lässt sich der Gehäuseoberabschnitt um das Scharnier 48 hochklappen (Figur 7). In dieser Position ist der mit der Verschlußtaste 47 in Verbindung stehende Arretierbolzen 49 erkennbar, der dafür sorgt, das der Gehäuseoberabschnitt 2a nur dann geöffnet werden kann, wenn die Druckfeder 16 gespannt ist. In dieser Position kann die Behälterpatrone 10 in die Aufnahmekammer 30 (gestrichelt) eingeschoben werden (entlang der gestrichelten Markierung).

Figur 8 zeigt eine Ausführungsform mit einem leicht L-förmigen Gehäuse 26 mit darauf befindlichem Mundstück 17. Ein Gelenkarm 27 erlaubt es, die mechanische Antriebseinheit 28 herauszuführen, und so die Behälterpatrone 10 in die gestrichelte Aufnahmekammer 30 einzuführen bzw. herauszunehmen. Nach einer entsprechenden Bestückung des Gerätes 1 mit die Behälterpatrone 10 wird der Gelenkarm 27 zurückgefahren, so daß das Gerät seine Betriebsstellung erreicht.

Gemäß Figur 9a zeigt eine Variante mit einem exzentrisch drehbar gelagerten Gehäuseoberabschnitt 2a. In diesem Fall kann der Behälter 10 erst dann in die Aufnahmekammer 30 eingebracht werden, wenn der Gehäuseoberabschnitt 2a seitlich, d.h. horizontal gegenüber dem Gehäusemittelabschnitt 2b herausgedreht wird. In dieser Position kann der Behälter 10 in die gestrichelte Position eingeführt werden, und dann das zusätzliche Teil zurückgeschoben werden.

Figur 9b zeigt in mehreren Momentaufnahmen von links nach rechts die Bestückung eines Gerätes mit einem Mechanismus, bei dem der Gehäuseoberabschnitt 2a nur dann geöffnet werden kann, wenn er zunächst vertikal gegen den Gehäusemittelabschnitt 2b bewegt wird.

Fig. 10 zeigt einen Längsschnitt durch ein Sperrspannwerk. Der obere zylindrische Gehäuseabschnitt 2, welches in diesem Fall ausschließlich den Gehäusemittelabschnitt 2b darstellt, Gehäuseoberabschnitt 2a ist nicht dargestellt, greift über das Druckfedergehäuse 31, mit dem es über Schnappnasen 32 verbunden ist. Die Schnappnasen 32 sind auf der Außenseite des Druckfedergehäuses 31 angebracht und erstrecken sich über zwei einander gegenüberliegende Kreissegmente von je etwa 30 Grad. Sie greifen in eine umlaufende Rille auf der Innenseite des oberen Gehäuseabschnitts 2 ein. Über das Druckfedergehäuse kann der Gehäuseunterabschnitt gestülpt werden und wird über Verbindungsmittel (Rastmittel) mit dem Druckfedergehäuse 31 abnehmbar, aber nicht gegeneinander drehbar verbunden (die Verbindung ist nicht dargestellt). Der Gehäusemittelabschnitt 2b bzw. der Gehäuseabschnitt 2 und das Druckfedergehäuse 31 sind gegeneinander drehbar. Durch die Verbindung des Druckfedergehäuses 16 mit dem Gehäuseunterabschnitt 3 sind auch die beiden Gehäuseabschnitte 2 und 3 und insbesondere 2b und 3 gegeneinander drehbar. Im Druckfedergehäuse 31 befindet sich die Druckfeder 16, die im allgemeinen bereits beim Zusammenstecken der beiden Gehäuseabschnitte vorgespannt wird. Die Druckfeder 16 stützt sich auf einem umlaufenden Vorsprung am unteren Ende des Druckfedergehäuses 31 ab sowie auf dem Abtriebsflansch 33, der zwischen dem oberen Gehäuseabschnitt 2 und dem Druckfedergehäuse 31 achsenparallel verschiebbar angeordnet ist und seinerseits gegen den oberen Gehäuseabschnitt 2 drückt. Der topfförmige Abtriebsflansch 33, der den Druckkolben 6 trägt, ragt in den oberen Gehäuseabschnitt 2 hinein. Das ringförmige Sperrglied 34 umschließt den Abtriebsflansch 33. Die am Sperrglied angebrachte Auslösetaste 35 ragt aus dem oberen Gehäuseabschnitt seitlich hervor.

Bei einem Schraubschubgetriebe enthält der Kragen des topfförmigen Abtriebsflansches 33 im allgemeinen zwei sägezahnförmige Aussparungen, auf denen zwei Sägezähne im oberen Gehäuseabschnitt abgleiten (nicht dargestellt). Beim Drehen des oberen Gehäuseabschnitts 2 gegen den Gehäuseunterabschnitt 3 und damit gegen das Druckfedergehäuse 31 wird der Abtriebsflansch 33 gegen die Kraft der Druckfeder 16 weiter in das Druckfedergehäuse 31 hineingedrückt. Sobald der obere Rand des Abtriebsflansches 33 durch das Sperrglied 34 hindurch weit genug nach unten gedrückt worden ist, schiebt sich das ringförmige Sperrglied 34 senkrecht zur Gehäuseachse zwischen den oberen Rand des Abtriebsflansches und einen ringförmigen Vorsprung 34 im oberen Gehäuseabschnitt 2 und hält den Abtriebsflansch 33 und die durch die Verschiebung des Abtriebsflansches zusätzlich gespannte Druckfeder 16 in der erreichten Position fest.

Die mittlere Druckfederkraft beträgt 10 bis 150 N. Zwischen der oberen und der unteren Ruhelage des Abtriebsteils ändert sich die Druckfederkraft etwa um ± 10 % der mittleren Druckfederkraft.

Durch Drücken der Auslösetaste 35 wird das ringförmige Sperrglied 34 senkrecht zur Gehäuseachse zurückgeschoben, wodurch der Weg des Abtriebsflansches 33 freigegeben wird. Die Druckfeder 16 schiebt den Abtriebsflansch 33 über eine vorgegebene Strecke nach oben und betätigt damit den mit dem Abtriebsflansch 33 verbundenes Druckkolben 6, der in dem Führungszylinder 38 geführt wird.

In Fig. 10 ist das Sperrspannwerk mit dem Abtriebsflansch 33 in seiner oberen Ruhelage und ausgerücktem Sperrglied 34 dargestellt. Fig. 11 zeigt das Sperrspannwerk mit dem Abtriebsflansch 33 in seiner unteren Ruhelage und eingerücktem Sperrglied 34. Der Anschlag 36 ist die Wegbegrenzung für das Abtriebsteil 33 in dessen unterer Ruhelage, der Anschlag 37 ist die Wegbegrenzung in dessen oberer Ruhelage. Durch Drehen der beiden Gehäuseabschnitte gegeneinander geht der Zustand nach Fig. 10 in den Zustand nach Fig. 11 über. Durch Drücken der Auslösetaste 35 geht der Zustand nach Fig. 11 in den Zustand nach Fig. 10 über.

Die Fig. 12 und 13 zeigen einen Querschnitt durch das Sperrspannwerk in Höhe der Mitte des ringförmigen Sperrgliedes, und zwar Fig. 12 entsprechend dem Zustand des Sperrspannwerkes nach Fig. 10 in ausgerückter Stellung des Sperrgliedes 34 und Fig. 13 entsprechend dem Zustand des Sperrspannwerkes nach Fig. 11 in eingerückter Stellung des Sperrgliedes 34.

Die Figuren 14a bis e zeigen die erfindungsgemäße Behälterpatrone 10. Kopfseitig befindet sich die Ausbringungseinrichtung 29, die eine Flüssigkeit leiten könnend mit dem Ausgang des Vorratszylinders 40 verbunden ist. Das bodenseitige Ende des Vorratszylinders 40 ist durch den Behälterkolben 39 verschlossen.
Die Öffnung der Ausbringungseinrichtung 29 ist durch eine obere Versiegelung 58 geschlossen. Der Behälterkolben 39 ist nach Außen durch die untere Versiegelung 59 geschlossen. Die Ausbringungseinrichtung 29 wird durch einen oder mehrer Halter 60 gehalten.
In Figur 14a ist der Halter 60 form- oder stoffschlüssig mit der Behälterpatrone 10 verbunden (z.B. verschweißt oder verklebt). In Figur 14b wird er durch eine Crimphülse 61 gehalten, in Figur 14c durch eine Überwurfmutter 62. In Figur 14e umgibt die Crimphülse 61 den Behälter vom Kopfbereich bis zum Boden.
In allen dargestellten Ausführungsformen ist die Bodenplatte 63 breiter als der Behälterbauch. Die Halterungen, wie Crimphülse oder Überwurfmutter sind dergestalt, daß sie die Öffnung der Ausbringungseinrichtung 29 frei geben, d.h. diese Öffnung nicht bedecken.

In allen figürlich dargestellten Ausführungsformen wird die Behälterpatrone bodenseitig in den Gehäuseoberabschnitt geschoben, bis die Bodenplatte 63 an die Randbegrenzung der bodenseitigen Öffnung der Aufnahmekammer 30 stößt.

Figur 15 zeigt einen Querschnitt durch die bevorzugte Düsenkonstruktion 41. Die Figur zeigt die reliefartige Mikrostruktur des Basisteils 42. Der Bereich 43 stellt den nicht geätzten Teil der Platte dar. Die Figur zeigt nur eine Düseöffnung 44 statt bevorzugt zwei gegeneinander geneigte Kanäle mit Düsenöffnungen. Die den zick-zack-konfigurierten Filter bildenden Vorsprünge tragen das Bezugszeichen 45. Die Düseneinlassseite trägt das Bezugszeichen 46.

Die Figur 16 zeigt im Querschnitt eine bevorzugte Ausführungsform des Geräts. Diese Darstellung weist das durch die Figuren 10 und 11 beschriebene Sperrspannwerk auf und weicht von der dort beschriebenen Vorrichtung nur geringfügig ab, insbesondere bei der Ausgestaltung des Druckkolbens 6 und des Abtriebsflansches 33. Gegenüber der Ausführungsform nach den Figuren 9 und 10 weist das Gerät in dieser Ausführungsform Blockiermittel auf, die Verhindern, das die Druckbeaufschlagung durch Drücken der Taste 35 ausgelöst werden kann, solange der Gehäuseoberabschnitt geöffnet ist. Diese Blockiermittel bestehen in dieser Ausführungsform aus einem Sperrbolzen 50 der bodenseitig gegen eine Feder 51 gelagert ist. Kopfseitig berührt der Sperrbolzen den Boden des Gehäuseoberabschnitts 2a. Der Sperrbolzen weist Bereiche mit größeren und kleineren Durchmessern auf. Er befindet sich hinter dem Sperrglied 34. Im geschlossenen Zustand des Geräts liegt eine am Sperrbolzen 50 ausgebildete Aussparung 52 hinter dem Sperrglied 34 und gibt dadurch den Weg für das Sperrglied frei. Im geöffneten Zustand des Geräts drückt die Feder 51 des Sperrbolzen leicht nach oben, so daß der breitere Bereich 53 des Sperrbolzens 50 hinter das Sperrglied 34 zu liegen kommt und damit die Freigabe des Sperrglieds blockiert. Ein Drücken der Auslösetaste 35 ist in diesem Zustand nicht möglich. In alternativen Ausführungsformen, ist die Feder 51 kopfseitig mit dem Sperrbolzen verbunden und der Mechanismus wird entsprechend gespiegelt. Figur 16 zeigt das geschlossene Gerät mit gespannter Druckfeder 16, d.h. der Kopf des Druckkolbens 6 liegt noch im Gehäusemittelabschnitt 2b.

Figur 17 zeigt eine andere Querschnittebene der Ausführungsform nach Figur 16 bei entspannter Feder. Der Kopf des Druckkolbens 6 hat den Behälterkolben 39 in die Behälterpatrone 10 hineingedrückt und die Flüssigkeit ist aus dieser durch die Düse 29 ausgebracht worden. In dieser Perspektive ist der Verschluß zwischen Gehäuseunterabschnitt und Druckfedergehäuse 31 unter dem Bezugszeichen 64 dargestellt. Der Verschluß kann lösbar oder fest sein, er kann über Schnappfeder u.ä. erreicht werden. Aus dieser Perspektive ist auch der Verschlußarretiermechanismus erkennbar. Am Gehäuseoberabschnitt 2a oder am Gehäusemittelabschnitt 2b ist die Verschlußtaste 54 ausgebildet. Diese berührt im geschlossenen Zustand des Geräts ein Ende des horizontal liegenden Arretierbolzens 55, der gegen die Feder 56 elastisch gelagert ist. Das andere Ende des Arretierbolzens liegt auf dem Druckkolben auf. In diesem Zustand kann die Verschlußtaste 54 nicht bedient werden. Erst wenn die Druckfeder 16 durch Drehen des Gehäuseunterabschnitts 3 gegen den Gehäuseinittelabschnitt 2b gespannt wird, wobei das Druckfedergehäuse 31 über den Verschluß 64 vom Gehäuseunterabschnitt mitgenommen wird, der Druckkolben 6 zurück in den Gehäusemittelabschnitt geführt wird, wird der Weg für den Arretierbolzen aus dieser Position heraus in Richtung zum Druckkolben freigegeben. In dieser Position kann die Verschlußtaste 54 gedrückt und der Gehäuseoberabschnitt geöffnet werden. In dieser Ausführungsform weist der Druckkolben einen stufenförmige Verjüngung 57 auf und der Arretierbolzen 55 wird durch den dickeren Bereich in seiner Bewegungsfreiheit blockiert. In anderen Ausführungsformen kann der Druckkolben 6 einen gleichbleibenden Durchmesser aufweisen und der Arretierbolzen 55 wird erst frei gegeben, wenn der Druckkolben durch die gespannte Druckfeder 16 unterhalb des Arretierbolzens gehalten wird.

Fig. 18a zeigt eine Aufnahmekammer, in die eine Behälterpatrone (10) mit einer Bodenplatte (63) mit einem Querschnitt in Form eines Kreissegments nach Fig. 18b. eingesetzt wird. Der Behälter verjüngt sich kopfseitig einmal stufenförmig. Die so ausgebildete Schulter wird von einer Verjüngung in der Aufnahmekammer gehalten.
Fig. 18c zeigt den selben Behälter mit dreieckiger Bodenplatte (63) und der Behälter nach
Fig. 18d weist eine quadratische Bodenplatte (63) auf.

In Fig. 18e ist ein Behälter (10) mit kreissegmentartigem Querschnitt aber runder Bodenplatte (63) nach Fig. 19f dargestellt, der sich in der dazu komplementären Aufnahmekammer befindet.

Die Figuren 19a und 19b demonstrieren eine Ausführungsform, die derart gestaltet ist, daß sich ein Verwender der vorliegenden Erfindung nicht verletzen kann, wenn die Behälterpatrone (10) aus Versehen von oben in eine Aufnahmekammer (30) eingeführt wird, für die die Zuführung der Behälterpatrone nur von unten vorgesehen ist. Fig. 19a zeigt eine Behälterpatrone (10), die von unten kommend in die Aufnahmekammer (30) eingeführt wurde. Das Gerät ist in gespanntem Zustand. Fig. 19b zeigt den Fall, bei dem die Behälterpatrone (10) von oben zugeführt wird. Das Gerät ist in entspanntem Zustand. Die Behälterpatrone (10) kann nur mit dem Kopfbereich in die Aufnahmekammer (30) geschoben, da dann die Schulter an die obere Kante der sich verjüngenden Aufnahmekammer (30) stößt. Der Druckkolben (6) reicht im entspannten Zustand des erfindungsgemäßen Apparats nicht so weit in die Aufnahmekammer (30) hinein, daß er die Behälterpatrone (10) berühren kann.

### Bezugszeichenliste

- 1: Vorrichtung zur Druckbeaufschlagung ggf. mit Behälter
- 2: Einheit aus Gehäuseoberabschnitt und Gehäusemittelabschnitt
- 2a: Gehäuseoberabschnitt
- 2b: Gehäusemittelabschnitt mit Sperrspannwerk
- 3: Gehäuseunterabschnitt
- 4: Öffnung
- 5: Symmetrieachse
- 6: Druckkolben
- 7: Schutzkappe
- 8a: Rastmechanismus
- 8b: Rastmechanismus
- 9: Austrittsöffnung für Flüssigkeit
- 10: Behälterpatrone
- 11: Nut
- 12: Halterung
- 13: verschiebbarer Knopf
- 14: Transportschlitten
- 15: Spannelement /Abtriebsflansch
- 16: Druckfeder
- 17: Mundstück
- 18: Griffstück
- 19: Scharnier vgl. 48
- 20: Rastnase
- 21: Schwenkklappe
- 22: Entriegelungsknopf
- 23: Verschiebemechanismus
- 24: zungenförmiger Abschnitt der Schutzkappe
- 25: zungenförmige Ausnehmung des Gehäuses
- 26: Gehäuse
- 27: Gelenkarm
- 28: mechanische Antriebseinheit
- 29: Zerstäubungseinrichtung
- 30: Aufnahmekammer
- 31: Druckfedergehäuse
- 32: Schnappnasen
- 33: Abtriebsflansch
- 34: Sperrglied
- 35: Auslösetaste vgl. 46
- 36: unterer Anschlag
- 37: oberer Anschlag
- 38: Führungszylinder
- 39: Behälterkolben
- 40: Vorratszylinder
- 41: Düsenkonstruktion
- 42: Basisteil
- 43: nicht geätzten Teil des Basisteils
- 44: Düseöffnung
- 45: Filter bildenden Vorsprünge
- 46: Düseneinlassseite
- 47: Verschlußtaste vgl. 54
- 48: Scharnier vgl. 19
- 49: Arretierbolzen
- 50: Sperrbolzen
- 51: Feder
- 52: Aussparung
- 53: dicker Bereich des Sperrbolzens
- 54: Verschlußtaste vgl. 47
- 55: Arretierbolzen
- 56: Feder
- 57: Verjüngung
- 58: obere Versiegelung
- 59: untere Versiegelung
- 60: Halter
- 61: Crimphülse
- 62: Überwurfmutter
- 63: Bodenplatte
- 64: Verschluß zwischen Gehäuseunterabschnitt und Druckfedergehäuse

## Patentansprüche

1. Vorrichtung zur Abgabe einer vordosierten Menge eines Arzneistoffes in gelöster oder suspendierter Form als Flüssigkeitsstrahl oder als Aerosol aus Tröpfchen durch Abgabe der vordosierten Menge unter Druck durch eine Ausbringungseinrichtung (29), umfassend
- ein elastisches Element (15,16) zur Speicherung einer vorbestimmten Energiemenge
- ein bewegliches Element (6), dem die vorbestimmte Energiemenge zuführbar ist, und welches die dosierte Fluidmenge einer vorbestimmten Druckerhöhung auszusetzen vermag,
- ein Mittel (4, 12, 13, 14, 18, 19, 21, 22, 23, 27) für das Einführen und Herausnehmen einer den Arzneistoff enthaltenden Behälterpatrone (10) in bzw. aus einer im Inneren der Vorrichtung liegenden Aufnahmekammer (30),
- ein Mittel für die Zuführung des druckbeaufschlagten Arzneistoffs zu einer mit der Behälterpatrone (10) fest verbundenen Ausbringungseinrichtung (29).
- eine Behälterpatrone (10), die über eine Öffnung (4) in liner Gehäusewand (26) der Vorrichtung in die Aufnahmekammer (30) einführbar ist, wobei die Behälterpatrone (10) direkt in ihre Endposition in der Vorrichtung einbringbar ist,
**dadurch gekennzeichnet, dass**
- die Behälterpatrone (10) nach ihrer Einführung in die Gehäuseöffnung (4) mittels eines Transportmittels, insbesondere eines Transportschlittens (14), in ihre Endposition überführbar ist.

2. Vorrichtung nach Anspruch 1, **dadurch gekennzeichnet, daß** ein Teil der Gehäusewand Bestandteil eines entfernbaren Griffstücks (18) ist, welches mit einer Halterung (12) zur Aufnahme einer Behälterpatrone (10) versehen ist.

3. Vorrichtung nach Anspruch 1 oder 2, **dadurch gekennzeichnet, daß** die Vorrichtung einen Gehäuseunterabschnitt (3) aufweist, dessen eines Ende das bodenseitige Ende der Vorrichtung definiert, einen gegen den Gehäuseunterabschnitt (3) drehbar gelagerten Gehäusemittelabschnitt (2b) und einen zum Gehäusemittelabschnitt (2b) vertikal klappbar oder exzentrisch drehbar ausgeführter Gehäuseoberabschnitt (2a) mit den Mitteln (30) zur Aufnahme der Behälterpatrone (10), wobei das im geschlossenen Zustand der Vorrichtung nicht mit dem Gehäusemittelabschnitt verbundene Ende das kopfseitige Ende der Vorrichtung definiert.

4. Vorrichtung nach Anspruch 63, **dadurch gekennzeichnet, daß** die Behälterpatrone (10) in eine durch den Gehäuseoberabschnitt (2a) durchgehende Bohrung (30) einführbar ist.

5. Vorrichtung nach einem Anspruch 4, **dadurch gekennzeichnet, daß** an der Bohrung (30) ein Anschlag oder mehrere Anschläge ausgebildet sind, über die der Behälter nicht hinweggeschoben werden kann und/oder Mittel zum Führen der Behälterpatrone (10) ggf. bis zu dem Anschlag bzw. den Anschlägen ausgebildet sind.

6. Vorrichtung nach Anspruch 1, **dadurch gekennzeichnet, daß** sich die Druckfeder (16) in einem Druckfedergehäuse (31) befindet, welches drehbar im Gehäusemittelabschnitt gelagert ist und mit dem Gehäuseunterabschnitt verbunden ist, wobei die Druckfeder (16) über ein Getriebe gespannt wird, wenn der Gehäuseunterabschnitt (3) und/oder das Druckfedergehäuse (31) gegen den Gehäusemittelabschnitt (2b) gedreht wird und dabei den Abtriebsflansch bodenseitig bewegt und die Druckfeder über ein Sperrglied (34) in der gespannten Position verharrt, bis ein Entspannung durch Drücken der mit dem Sperrglied (34) verbunden Auslösetaste (35) erfolgt.

7. Vorrichtung nach einem der Ansprüche 1 bis 6, **dadurch gekennzeichnet, daß** die Vorrichtung Verschlußarretiermittel (54, 55, 56) aufweist, die Verhindern, daß der Gehäuseoberabschnitt (2a) geöffnet werden kann, solange die Druckfeder (16) nicht gespannt ist und damit der Druckkolben (6) den Gehäuseoberabschnitt (2a) hineinragt.

8. Vorrichtung nach Anspruch 7 in Verbindung mit einem der Ansprüche 3 bis 6, **dadurch gekennzeichnet, daß** die Verschlußarretiermittel einen beweglichen Arretierbolzen (56) umfassen, der dem Auslösen der Versschlusstaste 54 solange im Weg steht, bis der Druckkolben (6) in der durch die gespannte Feder (16) definierten Position steht.

9. Formstabile, manuell nicht verformbare Behälterpatrone (10) mit Bodenteil und Kopfteil, wobei der Kopfteil durch eine Ausbringungseinrichtung (29) gebildet wird, von der aus ein Vorratszylinder (40) zur Aufnahme eines Arzneistoffes in gelöster oder suspendierter Form bis zum Boden der Behälterpatrone führt, der im Bodenbereich verschlossen ist durch eine nicht unzerstörbar von der Behälterpatrone entfernbare Versiegelung (59) und/oder einen in den Vorratszylinder (40) hinein bewegbaren, abdichtenden und nicht nach Außen über den Bodenbereich der Behälterpatrone hinausstehenden Behälterstempel (39) und/oder durch eine steife Bodenplatte (63).

10. Behälterpatrone nach Anspruch 9, **dadurch gekennzeichnet**, die Ausbringungseinrichtung (29) nach Außen hin durch eine Versiegelung (58) verschlossen ist.

11. Behälterpatrone nach Anspruch 9 oder 10. **dadurch gekennzeichnet, daß** die Ausbringungseinrichtung (29) durch wenigstens einen Halter (60) in der kopfseitigen Öffnung des Vorratzylinders (40) gehalten wird.

12. Behälterpatrone nach einem der Ansprüche 9 bis 11 **dadurch gekennzeichnet, daß** die Ausbringungseinrichtung (29) und /oder der Halter (60) durch verkleben, verschweißen, ultraverschweißen, vercrimpen und/oder eine Überwurfmutter in der kopfseitigen Öffnung des Vorratzylinders (40) gehalten wird (werden).

13. Behälterpatrone nach einem der Ansprüche 9 bis 12. **dadurch gekennzeichnet, daß** der Behälterstempel (39) ein Kolben (Behälterkolben) oder bevorzugt eine Kugel (Behälterkugel) ist.

14. Behälterpatrone nach einem der Ansprüche 9 bis 13, **dadurch gekennzeichnet, daß** der Vorratszylinder eine Füllvolumen von maximal 100 µl aufweist.

15. Behälterpatrone nach einem der Ansprüche 9 bis 14, **dadurch gekennzeichnet, daß** der Vorratszylinder eine Füllvolumen von maximal 15 µl aufweist.

16. Behälterpatrone nach einem der Ansprüche 9 bis 15, **dadurch gekennzeichnet, daß** Ausbringungseinrichtung (29) eine Düse mit einer Öffnung ist.

17. Behälterpatrone nach einem der Ansprüche 9 bis 15, **dadurch gekennzeichnet, daß** die Ausbringungseinrichtung (29) eine Düse mit wenigstens zwei Öffnungen ist.

18. Behälterpatrone nach Anspruch 17 **dadurch gekennzeichnet, daß** die zu den wenigstens zwei Öffnungen zuführenden Kanäle in Richtung der Öffnungen aufeinander zu ausgerichtet sind, so daß aus den Öffnungen ausgebrachte Flüssigkeitsstrahlen oder Aerosolwolken aufeinander prallen.

19. Behälterpatrone nach einem der Ansprüche 9 bis 18, **dadurch gekennzeichnet, daß** Ausbringungseinrichtung (29) Filtermittel (45) aufweist.

20. Behälterpatrone nach einem der Ansprüche 9 bis 19. **dadurch gekennzeichnet, daß** die Ausbringungseinrichtung (29) aus wenigstens zwei Teilen mit jeweils wenigstens einer, im wesentlichen ebenen Oberfläche besteht, über die die beiden Teile miteinander zu einer Einheit verbunden sind, wobei wenigstens eine der Oberflächen eine Mikrostruktur mit Kanälen aufweist, die zumindest einen Flüssigkeitseinlass in die Einheit und wenigstens einen Flüssigkeitsauslass aus der Einheit heraus, ggf. Filtermittel und/oder eine oder mehrere Plenumkammern bilden.

21. Behälterpatrone nach einem der Ansprüche 9 bis 20 **dadurch gekennzeichnet, daß** die Behälterpatrone plastisch nicht verformbar ist bis zu einem Druckunterschied zwischen dem Inneren des Vorratszylinders und der Außenumgebung von wenigstens 49 bar.

22. Behälterpatrone nach einem der Ansprüche 9 bis 21, **dadurch gekennzeichnet, daß** der Behälter einen Kopfbereich, eine Schulter und einen Bauchbereich aufweist, wobei der zur Längsachse vertikale Querschnitt des Bauchbereichs größer ist als der zur Längsachse vertikale Querschnitt des Kopfbereichs.

23. Behälterpatrone nach einem der Ansprüche 9 bis 22, **dadurch gekennzeichnet, daß** der Behälter eine Bodenplatte aufweist, die den vertikal zur Längsachse größten Querschnitt des Behälters aufweist.

24. Behälterpatrone nach einem der Ansprüche 9 bis 22, **dadurch gekennzeichnet, daß** ein Teil des Behälters einen bezüglich der Längsachse vertikalen Querschnitt aufweist, der nicht rotationssymmetrisch ist.

25. System zur Abgabe einer vordosierten Menge einer medizinisch-therapeutisch oder medizinisch-prophylaktisch wirksamen Substanz in gelöster oder suspendierter Form als ein Flüssigkeitsstrahl oder ein Aerosol aus Tröpfchen durch Abgabe der vordosierten Menge des Arzneistoffes unter Druck durch eine Ausbringungseinrichtung (29), umfassend eine Vorrichtung nach einem der Ansprüche 1 bis 8 sowie mindestens eine Behälterpatrone nach einem der Ansprüche 9 bis 24.

26. System nach Anspruch 25, **dadurch gekennzeichnet, daß** die Aufnahmekammer (30), und die Behälterpatrone (10) passgenau ausgebildet sind.

27. System nach Anspruch 25, **dadurch gekennzeichnet, daß** es sich um einen nadellosen Injektor handelt.

28. System nach Anspruch 25, **dadurch gekennzeichnet, daß** es sich um Inhaliergerät handelt.

29. System nach Anspruch 25, **dadurch gekennzeichnet, daß** es sich um einen Zerstäuber zum Aufbringen eines Sprays auf die Augenoberfläche handelt.

## Claims

1. Device for dispensing a pre-metered amount of a medicament in dissolved or suspended form as a jet of liquid or as an aerosol made up of droplets by dispensing the pre-metered amount under pressure through a delivery device (29), comprising
- a resilient element (15,16) for storing a predetermined amount of energy
- a movable element (6), to which the predetermined amount of energy can be supplied, and which is capable of exposing the metered amount of fluid to a predetermined pressure increase,
- a means (4, 12, 13, 14, 18, 19, 21, 22, 23, 27) for introducing and removing a container cartridge (10) that contains the medicament into or from a receiving chamber (30) located inside the device,
- a means for supplying the pressurised medicament to a delivery device (29) that is firmly attached to the container cartridge (10),
- a container cartridge (10) which can be introduced into the receiving chamber (30) through an opening (4) in a housing wall (26) of the device, wherein the
- container cartridge (10) can be placed directly in its final position in the device,
**characterised in that**
- after being introduced into the housing opening (4) the container cartridge (10) can be moved into its final position by transporting means, particularly a transporting slide (14).

2. Device according to claim 1, **characterised in that** a section of the housing wall is part of a removable handle (18) which is provided with a holder (12) for accommodating a container cartridge (10).

3. Device according to claim 1 or 2, **characterised in that** the device has a lower housing portion (3) one end of which defines the bottom end of the device, a central housing portion which is mounted to be rotatable counter to the lower housing portion (3), and an upper housing portion (2a) designed to be folded vertically or eccentrically rotated relative to the central housing portion (2b), having the means (30) for accommodating the container cartridge (10), while the end that is not connected to the central housing portion in the closed state of the device defines the head end of the device.

4. Device according to claim 3, **characterised in that** the container cartridge (10) can be inserted in a bore (30) passing through the upper housing portion (2a).

5. Device according to claim 4, **characterised in that** one or more abutments are formed on the bore (30), beyond which the container cannot be pushed, and/or means are formed for guiding the container cartridge (10) optionally as far as the abutment or abutments.

6. Device according to claim 1, **characterised in that** the compression spring (16) is located in a compression spring housing (31) which is rotatably mounted in the central housing portion and is connected to the lower housing portion, the compression spring (16) being tensioned by means of a gear when the lower housing portion (3) and/or the compression spring housing (31) is rotated counter to the central housing portion (2b) and the power take-off flange is moved at its base and the compression spring is held in the tensioned position by means of a locking member (34) until it is released by pressing the release button (35) connected to the locking member (34).

7. Device according to one of claims 1 to 6, **characterised in that** the device comprises closure locking means (54, 55, 56) which prevent the upper housing portion (2a) from being opened as long as the compression spring (16) is not under tension and as a result the compression piston (6) projects into the upper housing portion (2a).

8. Device according to claim 7 in conjunction with one of claims 3 to 6, **characterised in that** the closure locking means surround a movable locking bolt (56) which obstructs the actuation of the closure button 54 until the compression piston (6) is in the position defined by the tensioned spring (16).

9. Dimensionally stable, manually non-deformable container cartridge (10) having a base part and a head part, the head part being formed by a delivery device (29) from which a supply cylinder (40) for holding a medicament in dissolved or suspended form leads to the base of the container cartridge, which is sealed off at the base region by a seal (59) that cannot be removed from the container cartridge without being destroyed and/or a container ram (39) that is movable into the supply cylinder (40), forming a seal and not projecting outwards above the base region of the container cartridge, and/or by a rigid base plate (63).

10. Container cartridge according to claim 9, **characterised in that** the delivery device (29) is outwardly sealed by a seal (58).

11. Container cartridge according to claim 9 or 10, **characterised in that** the delivery device (29) is held by at least one holder (60) in the opening at the head end of the supply cylinder (40).

12. Container cartridge according to one of claims 9 to 11, **characterised in that** the delivery device (29) and /or the holder (60) is or are held by adhesion, welding, ultrawelding, crimping and/or by a check nut in the opening at the head end of the supply cylinder.

13. Container cartridge according to one of claims 9 to 12, **characterised in that** the container ram (39) is a piston (container piston) or preferably a ball (container ball).

14. Container cartridge according to one of claims 9 to 13, **characterised in that** the supply cylinder has a fill volume of not more than 100 µl.

15. Container cartridge according to one of claims 9 to 14, **characterised in that** the supply cylinder has a fill volume of not more than 15 µl.

16. Container cartridge according to one of claims 9 to 15, **characterised in that** the delivery device (29) is a nozzle with an opening.

17. Container cartridge according to one of claims 9 to 15, **characterised in that** the delivery device (29) is a nozzle with at least two openings.

18. Container cartridge according to claim 17, **characterised in that** the channels leading to the at least two openings are directed towards one another in the direction of the openings such that jets of liquid or aerosol mists delivered from the openings strike one another.

19. Container cartridge according to one of claims 9 to 18, **characterised in that** the delivery device (29) comprises filter means (45).

20. Container cartridge according to one of claims 9 to 19, **characterised in that** the delivery device (29) consists of at least two parts each having at least one substantially planar surface via which the two parts are joined together to form a unit, at least one of the surfaces having a microstructure with channels which form at least one liquid inlet into the unit and at least one liquid outlet out of the unit, optionally filter means and/or one or more plenum chambers.

21. Container cartridge according to one of claims 9 to 20, **characterised in that** the container cartridge is plastically non-deformable until there is a pressure difference between the inside of the supply cylinder and the outer environment of at least 49 bar.

22. Container cartridge according to one of claims 9 to 21, **characterised in that** the container has a head part, a shoulder and a belly part, the cross-section of the belly part which is vertical with respect to the longitudinal axis being greater than the cross-section of the head part which is vertical with respect to the longitudinal axis.

23. Container cartridge according to one of claims 9 to 22, **characterised in that** the container has a base plate which has the largest cross-section of the container vertically with respect to the longitudinal axis.

24. Container cartridge according to one of claims 9 to 22, **characterised in that** a part of the container has a vertical cross-section with respect to the longitudinal axis which is not rotationally symmetrical.

25. System for delivering a pre-metered amount of a medical-therapeutic or medical-preventative active substance in dissolved or suspended form as a jet of liquid or an aerosol made up of droplets by delivering the pre-metered amount of the medicament under pressure through a delivery device (29), comprising a device according to one of claims 1 to 8 and at least one container cartridge according to one of claims 9 to 24.

26. System according to claim 25, **characterised in that** the receiving chamber (30) and the container cartridge (10) are constructed so as to fit exactly,

27. System according to claim 25, **characterised in that** it is a needleless injector.

28. System according to claim 25, **characterised in that** it is an inhaler.

29. System according to claim 25, **characterised in that** it is an atomiser for applying a spray to the surface of the eye.

## Revendications

1. Dispositif servant à délivrer une quantité prédosée d'une substance médicamenteuse sous forme dissoute ou en suspension en tant que jet liquide ou en tant qu'aérosol de gouttelettes, un système de distribution (29) délivrant la quantité prédosée sous pression, comportant
■ un élément élastique (15, 16) servant à emmagasiner une quantité d'énergie prédéterminée,
■ un élément mobile (6), auquel la quantité d'énergie prédéterminée peut être amenée et qui est en mesure d'exposer la dose de quantité de fluide à une augmentation de pression prédéterminée,
■ un moyen (4, 12, 13, 14, 18, 19, 21, 22, 23, 27) pour l'introduction d'une cartouche de réservoir (10) contenant la substance médicamenteuse dans la chambre de réception (30) se trouvant à l'intérieur du dispositif et/ou pour son retrait hors de à chambre de réception,
■ un moyen pour amener la substance médicamenteuse sous pression à un système de distribution (29) relié de manière fixe à la cartouche de réservoir (10),
■ une cartouche de réservoir (10) qui peut être introduite dans la chambre de réception (30) via une ouverture (4) dans une paroi du boîtier (26) du dispositif, sachant que la cartouche de réservoir (10) peut être mise dans le dispositif directement dans sa position finale,
**caractérisé en ce que**
■ la cartouche de réservoir (10) peut être transférée après son introduction dans l'ouverture de boitier (4) dans sa position finale au moyen d'un moyen de transport, en particulier au moyen d'un traîneau de transport (14).

2. Dispositif selon la revendication 1, **caractérisé en ce qu'**une partie de la paroi du boîtier constitue un composant d'une pièce de préhension (18) pouvant être retirée, laquelle est munie d'une fixation (12) servant à recevoir une cartouche de réservoir (10).

3. Dispositif selon la revendication 1 ou 2, **caractérisé en ce que** le dispositif présente une section inférieure de boîtier (3) dont l'une des extrémités définit l'extrémité côté fond du dispositif, une section médiane de boîtier (2b) logée de manière à pouvoir tourner contre la section inférieure du boîtier (3) et une section supérieure du boîtier (2a) étant réalisée de manière à pouvoir tourner de manière excentrique ou de manière à pouvoir être rabattue verticalement par rapport à la section médiane du boîtier (2b) et comportant des moyens (30) servant à recevoir la cartouche de réservoir (10), sachant que l'extrémité n'étant pas reliée à la section médiane du boîtier lorsque le dispositif est fermé définit l'extrémité côté tête du dispositif.

4. Dispositif selon la revendication 3, **caractérisé en ce que** la cartouche du réservoir (10) peut être introduite dans un alésage (30) traversant la section supérieure de boîtier (2a).

5. Dispositif selon la revendication 4, **caractérisé en ce qu'**une butée ou plusieurs butées sont réalisées au niveau de l'alésage (30), au-delà desquelles le réservoir ne peut être poussé, et **en ce que** des moyens pour guider la cartouche de réservoir (10) le cas échéant jusqu'à la butée/ jusqu'aux butées sont réalisés.

6. Dispositif selon la revendication 1, **caractérisé en ce que** le ressort de pression (16) se trouve dans le boîtier du ressort de pression (31), lequel ressort est logé de manière à pouvoir tourner dans la section médiane de boîtier et est relié à la section inférieure du boîtier, sachant que le ressort de pression (16) est tendu par l'intermédiaire d'un engrenage, lorsque la section inférieure du boîtier (3) et/ou le boîtier du ressort de pression (31) est/sont tournés contre la section médiane du boîtier (2b) et ce faisant déplacent la bride de sortie côté du fond et lorsque le ressort de pression se bloque dans la position tendue au-dessus d'un membre de blocage (34) jusqu'à ce qu'il se produise une détente en pressant la touche de déclenchement (35) reliée au membre de blocage (34).

7. Dispositif selon l'une quelconque des revendications 1 à 6, **caractérisé en ce que** le dispositif présente des moyens d'arrêt par verrouillage (54, 55, 56), lesquels empêchent que la section supérieure du boîtier (2a) ne puisse s'ouvrir tant que le ressort de pression (16) n'est pas tendu et que le piston de pression (6) ne dépasse ainsi la section supérieure du boîtier (2a).

8. Dispositif selon la revendication 7 en liaison avec l'une quelconque des revendications 3 à 6, **caractérisé en ce que** les moyens d'arrêt par verrouillage comportent un piston d'arrêt (56) mobile, lequel s'oppose au déclenchement de la touche de verrouillage (54) tant que le piston de pression (6) se trouve dans la position définie par le ressort (16) tendu.

9. Cartouche de réservoir (10) présentant une forme stable, ne pouvant être déformée de manière manuelle, avec un fond et une tête, sachant que la tête est formée par un système de distribution (29), depuis lequel un cylindre de stockage (40), servant à recevoir une substance médicamenteuse sous une forme dissoute ou en suspension mène jusqu'au fond de la cartouche de réservoir, qui est fermé dans la zone du fond par un enduit de scellement (59) pouvant être retiré de manière non indestructible de la cartouche du réservoir et/ou par un poinçon du réservoir (39) pouvant être déplacé à l'intérieur du cylindre de stockage (40), étanchéifiant et ne pouvant dépasser à l'extérieur du fond de la cartouche de réservoir et/ou par une plaque de fond (63) inflexible.

10. Cartouche de réservoir selon la revendication 9, **caractérisée en ce que** le système de distribution (29) est fermé de l'extérieur par un enduit de scellement (58).

11. Cartouche de réservoir selon la revendication 9 ou 10, **caractérisée en ce que** le système de distribution (29) est maintenu par au moins un moyen de retenue (60) dans l'ouverture côté tête du cylindre de stockage (40).

12. Cartouche de réservoir selon l'une quelconque des revendications 9 à 11, **caractérisée en ce que** le système de distribution (29) et/ou le moyen de retenue (60) est/sont maintenu(s) dans l'ouverture côté tête du cylindre de stockage (40) par collage, soudage, soudage à ultrasons, sertissage et/ou par un écrou-raccord.

13. Cartouche de réservoir selon l'une quelconque des revendications 9 à 12, **caractérisée en ce que** le poinçon du réservoir (39) est un piston (piston de réservoir) ou de préférence une bille (bille de réservoir).

14. Cartouche de réservoir selon l'une quelconque des revendications 9 à 13, **caractérisée en ce que** le cylindre de stockage présente un volume maximal de remplissage de 100 µl.

15. Cartouche de réservoir selon l'une quelconque des revendications 9 à 14, **caractérisée en ce que** le cylindre de stockage présente un volume maximal de remplissage de 15 µl.

16. Cartouche de réservoir selon l'une quelconque des revendications 9 à 15, **caractérisée en ce que** le système de distribution (29) est une buse avec une ouverture.

17. Cartouche de réservoir selon l'une quelconque des revendications 9 à 15, **caractérisée en ce que** le système de distribution (29) est une buse avec au moins deux ouvertures.

18. Cartouche de réservoir selon la revendication 17, **caractérisée en ce que** les canaux menant à au moins deux ouvertures sont orientés en direction des ouvertures de sorte que les jets liquides sortant des ouvertures ou les nuages aérosol s'entrechoquent.

19. Cartouche de réservoir selon l'une quelconque des revendications 9 à 18, **caractérisée en ce que** le système de distribution (29) présente des moyens de filtrage (45).

20. Cartouche de réservoir selon l'une quelconque des revendications 9 à 19, **caractérisée en ce que** le système de distribution (29) se compose d'au moins deux parties avec respectivement au moins une surface essentiellement plate, au-dessus de laquelle les deux parties sont reliées entre elles pour former une unité, au moins une des surfaces présentant une microstructure avec des canaux, lesquels forment au moins une entrée de liquide dans l'unité et au moins une sortie de liquide de l'unité, le cas échéant des moyens de filtrage et/ou une ou plusieurs chambres d'accumulation.

21. Cartouche de réservoir selon l'une quelconque des revendications 9 à 20, **caractérisée en ce que** la cartouche de réservoir ne peut être déformée de manière plastique jusqu'à une différence de pression d'au moins 49 bar entre l'intérieur du cylindre de stockage et l'environnement extérieur.

22. Cartouche de réservoir selon l'une quelconque des revendications 9 à 21, **caractérisée en ce que** le réservoir présente une zone de tête, une épaule et une zone de ventre, la section transversale de la zone de ventre, verticale par rapport à l'axe longitudinal, étant plus grande que la section transversale de la zone de tête verticale par rapport à l'axe longitudinal.

23. Cartouche de réservoir selon l'une quelconque des revendications 9 à 22, **caractérisée en ce que** le réservoir présente une plaque de fond, qui présente la section transversale la plus grande de manière verticale par rapport à l'axe longitudinal.

24. Cartouche de réservoir selon l'une quelconque des revendications 9 à 22, **caractérisé en ce qu'**une partie du réservoir présente une section transversale verticale par rapport à l'axe longitudinal, laquelle n'est pas symétrique par rotation.

25. Système servant à délivrer une quantité prédosée d'une substance active d'un point de vue médico-thérapeutigue ou médico-prophylactique sous une forme dissoute ou en suspension comme un jet liquide ou un aérosol de gouttelettes, un système de distribution (29) délivrant une quantité prédosée d'une substance médicale sous pression, comportant un dispositif selon l'une quelconque des revendications 1 à 8 ainsi qu'au moins une cartouche de réservoir selon l'une quelconque des revendications 9 à 24.

26. Système selon la revendication 25, **caractérisé en ce que** les chambres de réception (30) et la cartouche de réservoir (10) sont réalisées de manière à s'adapter l'un à l'autre.

27. Système selon la revendication 25, **caractérisé en ce qu'**il s'agit d'un appareil à injection sans aiguille.

28. Système selon la revendication 25, **caractérisé en ce qu'**il s'agit d'un appareil d'inhalation.

29. Système selon la revendication 25, **caractérisé en ce qu'**il s'agit d'un pulvérisateur servant à appliquer un spray à la surface des yeux.
